# EUROPÄISCHE PATENTSCHRIFT

(11) **EP 2 192 115 B1**
(45) Veröffentlichungstag und Bekanntmachung des Hinweises auf die Patenterteilung: **12.02.2014**
(21) Anmeldenummer: 10152240.7
(22) Anmeldetag: 12.10.2004
(51) Int. Cl.: C07D 231/14, C07D 277/56, C07D 231/16, C07D 333/38, C07D 307/68, C07D 327/06, C07D 213/82, A01N 43/56, A01N 43/40, A01N 43/78, A01N 43/32, A01N 43/08, A01N 43/10

(54) **1,3-DIMETHYLBUTYLCARBOXANILIDE ZUR BEKÄMPFUNG VON UNERWÜNSCHTEN MIKRORGANISMEN**
1,3-DIMETHYLBUTYLCARBOXANILIDES FOR CONTROLLING UNDESIRABLE MICRO-ORGANISMS
1,3-DIMÉTHYLBUTYLCARBOXANILIDES POUR CONTRÔLER DES MICRO-ORGANISMES INDÉSIRABLES

(30) Priorität: 23.10.2003 DE 10349502
(43) Veröffentlichungstag der Anmeldung: 02.06.2010
(62) Teilanmeldung aus: 04765931.3
(73) Patentinhaber: Bayer CropScience AG, 40789 Monheim (DE)
(72) Erfinder: Dunkel, Ralf, Dr., 42799 Leichlingen (DE); Elbe, Hans-Ludwig, Dr., 42329 Wuppertal (DE); Greul, Jörg Nico, Dr., 42799 Leighlingen (DE); Hartmann, Benoit, 69110 Ste Foy les Lyons (FR); Wachendorff-Neumann, Ulrike, Dr., 56566 Neuwied (DE); Dahmen, Peter, Dr., 41470 Neuss (DE); Kuck, Karl-Heinz, Dr., 40764 Langenfeld (DE)

(56) Entgegenhaltungen:
- EP-A- 0 824 099
- WO-A-02/059086
- WO-A-02/096882
- WO-A2-2005/041653
- WO-A2-2005/122770
- PATENT ABSTRACTS OF JAPAN Bd. 2000, Nr. 20, 10. Juli 2001 (2001-07-10) & JP 2001 072507 A (MITSUI CHEMICALS INC), 21. März 2001 (2001-03-21)

## Beschreibung

Die vorliegende Erfindung betrifft neue 1,3-Dimethylbutylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vgl. z.B. WO 03/010149, WO 02/059086, WO 02/38542, EP-A 0 824 099, EP-A 0 591 699, EP-A 0 589 301, EP-A 0 545 099, JP 11-335364 und JP 10-251240), wie z.B. N-[2-(1,3-Dimethylbutyl)phenyl]-5-fluor-1,3-dimethyl-1H-pyrazol-4-carboxamid (WO 03/010149), N-Allyl-N-[2-(1,3-dimethylbutyl)phenyl]-1-methyl-3-(trifluormethyl)-1H-pyrazol-4-carboxamid (WO 02/059086), N-[2-(1,3-Dimethylbutyl)phenyl]-1-methyl-4-(trifluormethyl)-1H-pyrrol-3-carboxamid (WO 02/38542), N-[2-(1,3-Dimethylbutyl)-phenyl]-2-methyl-4,5-dihydrofuran-3-carboxamid (JP 11-335364). Die Wirksamkeit dieser Stoffe ist gut, lässt aber bei niedrigen Aufwandmengen in manchen Fällen zu wünschen übrig.

Es wurden nun neue 1,3-Dimethylbutylcarboxanilide der Formel (I) 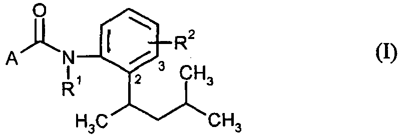 gefunden, in welcher
- R¹: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkyl-sulfinyl, C₁-C₄-Halogenalkylsulfonyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
oder -C(=O)C(=O)R³, -CONR⁴R⁵ oder -CH₂NR⁶R⁷ steht,
- R²: für Wasserstoff steht,
- R³: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R⁴ und R⁵: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen stehen,
- R⁴ und R⁵: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁸ enthalten kann,
- R⁶ und R⁷: unabhängig voneinander für Wasserstoff, C₁-C₈-Alkyl, C₃-C₈-Cycloalkyl; C₁-C₈-Halogen-alkyl, C₃-C₈-Halogencycloalkyl mit jeweils 1 bis 9 Fluor-, Chlor- und/oder Bromatomen stehen,
- R⁶ und R⁷: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁸ enthalten kann,
- R⁸: für Wasserstoff oder C₁-C₆-Alkyl steht,
- A: für den Rest der Formel (A1) 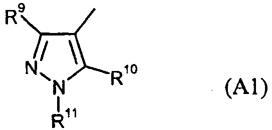 steht, in welcher
R⁹ für Fluor, Chlor, Brom, oder C₂-Halogenalkyl mit 1 bis 5 Halogenatomensteht,
R¹⁰ für Wasserstoff, Chlor, Brom, Iod, Cyano, C₁-C₄-Alkyl, C₁-C₄-Alkoxy, C₁-C₄-Alkylthio oder C₁-C₄-Halogenalkyl mit 1 bis 5 Halogenatomen steht und
R¹¹ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht,
oder
- A: für den Rest der Formel (A2) 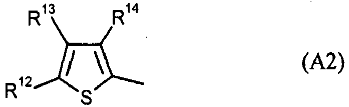 (A2) steht, in welcher
- R¹² und R¹³: unabhängig voneinander für Wasserstoff, Halogen, C₁-C₄-Alkyl oder C₁-C₄-Halogenalkyl mit jeweils 1 bis 5 Halogenatomen stehen und
- R¹⁴: für Halogen, Cyano oder C₁-C₄-Alkyl, oder C₁-C₄-Halogenalkyl oder C₁-C₄-Halogenalkoxy mit jeweils 1 bis 5 Halogenatomen steht, mit der Maßgabe, dass R¹⁴ nicht für Methyl steht, wenn R¹² und R¹³ für Wasserstoff oder Methyl stehen und R¹ und R² gleichzeitig für Wasserstoff stehen,
oder
- A: für den Rest der Formel (A3) 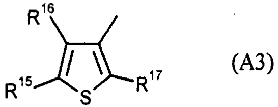 (A3) steht, in welcher
R¹⁵ und R¹⁶ unabhängig voneinander für Wasserstoff, Halogen, C₁-C₄-Alkyl oder C₁-C₄-Halogenalkyl mit 1 bis 5 Halogenatomen stehen und
R¹⁷ für Wasserstoff, C₁-C₄-Alkyl oder C₁-C₄-Halogenalkyl mit 1 bis 5 Halogenatomen steht,
oder
- A: für den Rest der Formel (A4) 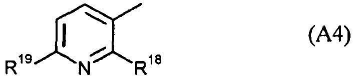 (A4) steht, in welcher
R¹⁸ für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R¹⁹ für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulfinyl oder C₁-C₄-Alkylsulfonyl steht,
mit der Maßgabe,
a) dass R¹⁸ nicht für Trifluormethyl, Methyl, Chlor oder Methylthio steht, wenn R¹⁹ für Wasserstoff steht und R¹ und R² gleichzeitig für Wasserstoff stehen,
oder
- A: für den Rest der Formel (A5) 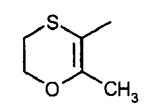 steht,
mit der Maßgabe, dass R¹ und R² nicht gleichzeitig für Wasserstoff stehen, wenn A für A5 steht,
oder
- A: für den Rest der Formel (A6) 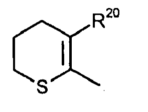 steht, in welcher
R²⁰ für C₁-C₄-Alkyl oder C₁-C₄-Halogenalkyl mit 1 bis 5 Halogenatomen steht,
oder
- A: für den Rest der Formel (A7) 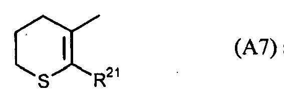 steht, in welcher
R²¹ für C₁-C₄-Alkyl oder C₁-C₄-Halogenalkyl mit 1 bis 5 Halogenatomen steht,
oder
- A: für den Rest der Formel (A8) 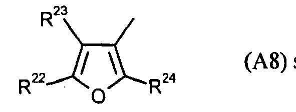 steht, in welcher
R²² und R²³ unabhängig voneinander für Wasserstoff, Halogen, Amino, C₁-C₄-Alkyl oder C₁-C₄-Halogenalkyl mit 1 bis 5 Halogenatomen steht und
R²⁴ für Wasserstoff, C₁-C₄-Alkyl oder C₁-C₄-Halogenalkyl mit 1 to 5 Halogenatomen steht,
mit der Maßgabe, dass R²⁴ nicht für Methyl steht, wenn R²² und R²³ für Wasserstoff oder Methyl stehen und R¹ und R² gleichzeitig für Wasserstoff stehen,
oder
- A: für den Rest der Formel (A9) 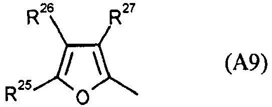 steht, in welcher
- R²⁵ und R²⁶: unabhängig voneinander für Wasserstoff, Halogen, Amino, Nitro, C₁-C₄-Alkyl oder C₁-C₄-Halogenalkyl mit 1 bis 5 Halogenatomen stehen und
- R²⁷: für Halogen, C₁-C₄-Alkyl oder C₁-C₄-Halogenalkyl mit 1 bis 5 Halogenatomen steht,
oder
- A: für den Rest der Formel (A11) 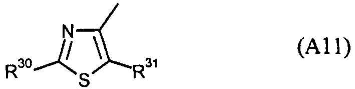 ) steht, in welcher
R³⁰ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht und
R³¹ für Halogen, C₁-C₄-Alkyl oder C₁-C₄-Halogenalkyl mit 1 bis 5 Halogenatomen steht,
oder
- A: für den Rest der Formel (A12) 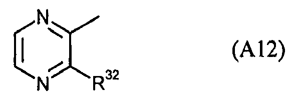 steht, in welcher
- R³²: für Wasserstoff, Halogen, C₁-C₄-Alkyl oder C₁-C₄-Halogenalkyl mit 1 bis 5 Halogenatomen steht,
mit der Maßgabe, dass R³² nicht für Chlor steht, wenn R¹ und R² gleichzeitig für Wasserstoff stehen,
oder
- A: für den Rest der Formel (A13) 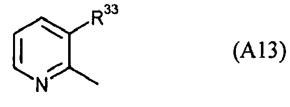 steht, in welcher
R³³ für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
- A: für den Rest der Formel (A14) 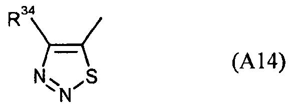 steht, in welcher
R³⁴ für C₁-C₄-Alkyl steht.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Weiterhin wurde gefunden, dass man 1,3-Dimethylbutylcarboxanilide der Formel (I) erhält, indem man
a) Carbonsäure-Derivate der Formel (II) 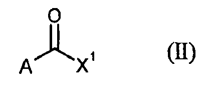 in welcher
   - A: die oben angegebenen Bedeutungen hat und
   - X¹: für Halogen oder Hydroxy steht,
   mit Anilin-Derivaten der Formel (III) 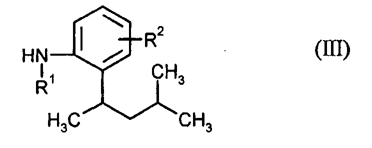 in welcher R¹ und R² die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
b) Hexylcarboxanilide der Formel (I-a) 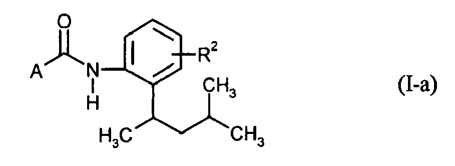 in welcher A und R² die oben angegebenen Bedeutungen haben
   mit Halogeniden der Formel (IV) 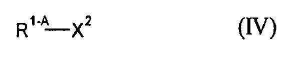 in welcher
   - X²: für Chlor, Brom oder Iod steht,
   - R¹: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; oder -C(=O)C(=O)R³, CONR⁴R⁵ oder -CH₂NR⁶R⁷ steht, wobei R³, R⁴, R⁵, R⁶ und R⁷ die oben angegebenen Bedeutungen haben,
in Gegenwart einer Base und in Gegenwart eines Verdünnungsmittels umsetzt.

Schließlich wurde gefunden, dass die neuen 1,3-Dimethylbutylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen 1,3-Dimethylbutylcarboxanilide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht bevorzugt für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R³, -CONR⁴R⁵ oder -CH₂NR⁶R⁷_{.}
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CC₃;
Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R³, -CONR⁴R⁵ oder -CH₂NR⁶R⁷.
- R¹: steht ganz besonders bevorzugt für Wasserstoff, Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂. -C(=O)CHO, -C(=O)C(=O)CH₃, -C(=O)C(=O)CH₂OCH₃, -C(=O)CO₂CH₃, -C(=O)CO₂CH₂CH₃.
- R²: steht bevorzugt für Wasserstoff.
- R³: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R³: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluormethyl, Trifluormethoxy.
- R⁴ und R⁵: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁴ und R⁵: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 oder 6 Ringatomen, wobei der Hetero-cyclus 1 oder 2 weitere, nicht benachbarte Heteroatome aus der Reihe Sauerstoff, Schwefel oder NR⁸ enthalten kann.
- R⁴ und R⁵: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Tri-fluorethyl, Trifluormethoxymethyl.
- R⁴ und R⁵: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁸ substituiert sein kann.
- R⁶ und R⁷: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R⁶ und R⁷: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁸ enthalten kann.
- R⁶ und R⁷: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁶ und R⁷: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁸ substituiert sein kann.
- R⁸: steht bevorzugt für Wasserstoff oder C₁-C₄-Alkyl.
- R⁸: steht besonders bevorzugt für Wasserstoff, Methyl, Ethyl, n- oder iso-Propyl, n-, iso-, sec- oder tert-Butyl.
- A: steht bevorzugt für einen der oben angegebenen Reste
A1, A2, A3, A4, A5, A8, A9, A10, A11, A12 oder A13.
- A: steht besonders bevorzugt für einen der oben angegebenen Reste A1, A2, A4, A5, A8, A12 oder A13.
- A: steht ganz besonders bevorzugt für den Rest A1.
- A: steht außerdem ganz besonders bevorzugt für den Rest A2.
- A: steht außerdem ganz besonders bevorzugt für den Rest A4.
- A: steht außerdem ganz besonders bevorzugt für den Rest A5. A steht außerdem ganz besonders bevorzugt für den Rest A8.
- A: steht außerdem ganz besonders bevorzugt für den Rest A 12.
- A: steht außerdem ganz besonders bevorzugt für den Rest A13.
- R⁹: steht bevorzugt für Fluor, Chlor, Brom, C₂-Halogenalkyl, mit 1 bis 5 Fluor, Chlor und/oder Bromatomen, , mit der Maßgabe,
a) dass R⁹ nicht für Chlor oder Brom steht, wenn R¹⁰ für Wasserstoff, Trifluormethyl oder Methyl steht, R¹¹ für Methyloder Trifluormethyl, steht und R¹ für (C₁-C₆-Alkyl)carbonyl, (C₁-C₆-Alkoxy)carbonyl, (C₁-C₄-Alkoxy-C₁-C₄-alkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl mit jeweils 1 bis 9 Fluor-, Chlor- und/ oder Bromatomen steht.
- R⁹: steht besonders bevorzugt für Fluor, Chlor, Brom, Monofluorethyl, Pentafluorethyl,,
- R⁹: steht ganz besonders bevorzugt für Fluor, Chlor, Brom, -CHFCH₃, Pentafluorethyl, , mit der Maßgabe,
- R⁹: steht insbesondere bevorzugt für Chlor, -CHFCH₃,
- R¹⁰: steht bevorzugt für Wasserstoff, Chlor, Brom, Iod, Methyl, Ethyl, Methoxy, Ethoxy, Methylthio, Ethylthio oder C₁-C₂-Halogenalkyl mit 1 bis 5 Halogenatomen.
- R¹⁰: steht besonders bevorzugt für Wasserstoff, Chlor, Brom, Iod, Methyl oder -CHFCH₃.
- R¹⁰: steht ganz besonders bevorzugt für Wasserstoff, Chlor, Methyl oder-CHFCH₃.
- R¹¹: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹¹: steht besonders bevorzugt für Wasserstoff, Methyl, Ethyl, iso-Propyl, Trifluormethyl, Difluormethyl, Hydroxymethyl, Hydroxyethyl oder Phenyl.
- R¹¹: steht ganz besonders bevorzugt für Wasserstoff, Methyl, Trifluormethyl oder Phenyl.
- R¹¹: steht insbesondere bevorzugt für Methyl.
- R¹² und R¹³: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹² und R¹³: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹² und R¹³: stehen unabhängig voneinander ganz besonders bevorzugt für Wasserstoff, Fluor, Chlor, Brom oder Methyl.
- R¹² und R¹³: stehen insbesondere bevorzugt jeweils für Wasserstoff.
- R¹⁴: steht bevorzugt für Fluor, Chlor, Brom, Iod, Cyano, Methyl, Ethyl, C₁-C₂-Halogenalkyl oder C₁-C₂-Halogenalkoxy mit jeweils 1 bis 5 Fluor, Chlor und/oder Bromatomen, mit der Maßgabe, dass R¹⁴ nicht für Methyl steht, wenn R¹² und R¹³ für Wasserstoff oder Methyl stehen und R¹ und R² gleichzeitig für Wasserstoff stehen.
- R¹⁴: steht besonders bevorzugt für Fluor, Chlor, Brom, Iod, Cyano, Methyl, Trifluormethyl, Trifluormethoxy, Difluormethoxy, Difluorchlormethoxy oder Trichlormethoxy, mit der Maßgabe, dass R¹⁴ nicht für Methyl steht, wenn R¹² und R¹³ für Wasserstoff oder Methyl stehen und R¹ und R² gleichzeitig für Wasserstoff stehen.
- R¹⁴: steht ganz besonders bevorzugt für Fluor, Chlor, Brom, Iod, Methyl, Trifluormethyl oder Trifluormethoxy, mit der Maßgabe, dass R¹⁴ nicht für Methyl steht, wenn R¹² und R¹³ für Wasserstoff oder Methyl stehen und R¹ und R² gleichzeitig für Wasserstoff stehen.
- R¹⁴: steht insbesondere bevorzugt für Chlor, Iod oder Methyl, mit der Maßgabe, dass R¹⁴ nicht für Methyl steht, wenn R¹² und R¹³ für Wasserstoff oder Methyl stehen und R¹ und R² gleichzeitig für Wasserstoff stehen.
- R¹⁵ und R¹⁶: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁵ und R¹⁶: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁵ und R¹⁶: stehen unabhängig voneinander ganz besonders bevorzugt für Wasserstoff, Fluor, Chlor, Brom oder Methyl.
- R¹⁵ und R¹⁶: stehen insbesondere bevorzugt jeweils für Wasserstoff.
- R¹⁷: steht bevorzugt für Wasserstoff, Methyl, Ethyl oder C₁-C₂-Halogenalkyl mit 1 bis 5 Fluor, Chlor und/oder Bromatomen.
- R¹⁷: steht besonders bevorzugt für Wasserstoff, Methyl oder Trifluormethyl.
- R¹⁷: steht ganz besonders bevorzugt für Methyl.
- R¹⁸: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen mit der Maßgabe,
a) dass R¹⁸ nicht für Trifluormethyl, Methyl, Chlor oder Methylthio steht, wenn R¹⁹ für Wasserstoff steht,
b) dass R¹⁸ nicht für Methyl, Difluorchlormethyl, Trifluormethyl, Difluormethyl, Chlor oder Brom steht, wenn R¹⁹ für Wasserstoff steht und R¹ für (C₁-C₆-Alkyl)carbonyl, (C₁-C₆-Alkoxy)carbonyl, (C₁-C₄-Alkoxy-C₁-C₄-alkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)-carbonyl mit jeweils 1 bis 9 Fluor-, Chlor- und/oder Bromatomen steht.
- R¹⁸: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluormethylthio, Trifluormethylthio, Trifluormethoxy, Difluormethoxy, Difluorchlormethoxy oder Trichlormethoxy
mit der Maßgabe,
a) dass R¹⁸ nicht für Trifluormethyl, Methyl, Chlor oder Methylthio steht, wenn R¹⁹ für Wasserstoff steht,
- R¹⁸: steht ganz besonders bevorzugt für Fluor, Chlor, Brom, Iod, Methyl, Trifluormethyl, Difluormethyl oder Trichlormethyl mit der Maßgabe,
a) dass R¹⁸ nicht für Trifluormethyl, Methyl, Chlor oder Methylthio steht, wenn R¹⁹ für Wasserstoff steht,
- R¹⁹: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulfinyl oder C₁-C₂-Alkylsulfonyl.
- R¹⁹: steht besonders bevorzugt für Wasserstoff, Fluor, Chlor, Brom, Iod, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy, Trichlormethoxy, Methylsulfinyl oder Methylsulfonyl.
- R¹⁹: steht ganz besonders bevorzu2:t für Wasserstoff, Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Trichlormethyl, Methylsulfinyl oder Methylsulfonyl.
- R¹⁹: steht insbesondere bevorzugt für Wasserstoff.
- R²⁰: steht bevorzugt für Methyl, Ethyl oder C₁-C₂-Halogenalkyl mit 1 bis 5 Fluor, Chlor und/oder Bromatomen.
- R²⁰: steht besonders bevorzugt für Methyl, Trifluormethyl, Difluormethyl, Difluorchlormethyl oder Trichlormethyl.
- R²¹: steht bevorzugt für Methyl, Ethyl oder C₁-C₂-Halogenalkyl mit 1 bis 5 Fluor, Chlor und/oder Bromatomen.
- R²¹: steht besonders bevorzugt für Methyl, Trifluormethyl, Difluormethyl, Difluorchlormethyl oder Trichlormethyl.
- R²² und R²³: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R²² und R²³: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R²² und R²³: stehen unabhängig voneinander ganz besonders bevorzugt für Wasserstoff, Fluor, Chlor, Brom oder Methyl.
- R²² und R²³: stehen insbesondere bevorzugt jeweils für Wasserstoff.
- R²⁴: steht bevorzugt für Wasserstoff, Methyl, Ethyl oder C₁-C₂-Halogenalkyl mit 1 bis 5 Fluor, Chlor und/oder Bromatomen, mit der Maßgabe, dass R²⁴ nicht für Methyl steht, wenn R²² und R²³ für Wasserstoff oder Methyl stehen und R¹ und R² gleichzeitig für Wasserstoff stehen.
- R²⁴: steht besonders bevorzugt für Wasserstoff, Methyl, Ethyl, Trifluormethyl, Difluormethyl, Difluorchlormethyl oder Trichlormethyl, mit der Maßgabe, dass R²⁴ nicht für Methyl steht, wenn R²² und R²³ für Wasserstoff oder Methyl stehen und R¹ und R² gleichzeitig für Wasserstoff stehen.
- R²⁴: steht ganz besonders bevorzugt für Wasserstoff, Methyl, Trifluormethyl, Difluormethyl oder Trichlormethyl, mit der Maßgabe, dass R²⁴ nicht für Methyl steht, wenn R²² und R²³ für Wasserstoff oder Methyl stehen und R¹ und R² gleichzeitig für Wasserstoff stehen.
- R²⁴: steht insbesondere bevorzugt für Methyl oder Trifluormethyl, mit der Maßgabe, dass R²⁴ nicht für Methyl steht, wenn R²² und R²³ für Wasserstoff oder Methyl stehen und R¹ und R² gleichzeitig für Wasserstoff stehen.
- R²⁵ und R²⁶: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Brom-atomen.
- R²⁵ und R²⁶: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl.
- R²⁵ und R²⁶: stehen unabhängig voneinander ganz besonders bevorzugt für Wasserstoff, Fluor, Chlor, Brom oder Methyl.
- R²⁵ und R²⁶: stehen insbesondere bevorzugt jeweils für Wasserstoff.
- R²⁷: steht bevorzugt für Fluor, Chlor, Brom, Methyl, Ethyl oder C₁-C₂-Halogenalkyl mit 1 bis 5 Fluor, Chlor und/oder Bromatomen.
- R²⁷: steht besonders bevorzugt für Fluor, Chlor, Brom, Methyl, Ethyl, Trifluormethyl, Difluormethyl, Difluorchlormethyl oder Trichlormethyl.
- R²⁷: steht ganz besonders bevorzugt für Fluor, Chlor, Brom, Methyl, Trifluormethyl, Difluormethyl oder Trichlormethyl.
- R²⁷: steht insbesondere bevorzugt für Methyl.
- R²⁸: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R²⁸: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R²⁸: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R²⁸: steht insbesondere bevorzugt für Wasserstoff, Chlor, Amino, Methylamino, Dimethylamino, Methyl oder Trifluormethyl.
- R²⁹: steht bevorzugt für Fluor, Chlor, Brom, Hydroxy, Methyl, Ethyl, Methoxy, Ethoxy, Cyclopropyl oder C₁-C₂-Halogenalkyl oder C₁-C₂-Halogenalkoxy mit jeweils 1 bis 5 Fluor, Chlor und/oder Bromatomen, mit der Maßgabe,
a) dass R²⁹ nicht für Trifluormethyl, Difluormethyl, Methyl oder Ethyl, wenn R²⁸ für Wasserstoff oder Methyl steht und R¹ und R² gleichzeitig für Wasserstoff stehen,
b) dass R²⁹ nicht für Methyl, Difluorchlormethyl, Trifluormethyl, Difluormethyl, Chlor oder Brom steht, wenn R¹¹ für Methyl, Trifluormethyl, Methoxymethyl oder Trifluormethoxymethyl steht und R¹ für (C₁-C₆-Alkyl)carbonyl, (C₁-C₆-Alkoxy)carbonyl, (C₁-C₄-Alkoxy-C₁-C₄-alkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl mit jeweils 1 bis 9 Fluor-, Chlor- und/oder Bromatomen steht.
- R²⁹: steht besonders bevorzugt für Fluor, Chlor, Brom, Hydroxy, Methyl, Ethyl, Methoxy, Ethoxy, Cyclopropyl, Trifluormethyl, Difluormethyl, Difluorchlormethyl, Trichlormethyl, Trifluormethoxy oder Difluormethoxy,
mit der Maßgabe,
a) dass R²⁹ nicht für Trifluormethyl, Difluormethyl, Methyl oder Ethyl, wenn R²⁸ für Wasserstoff oder Methyl steht und R¹ und R² gleichzeitig für Wasserstoff stehen,
b) dass R²⁹ nicht für Methyl, Difluorchlormethyl, Trifluormethyl, Difluormethyl, Chlor oder Brom steht, wenn R¹¹ für Methyl, Trifluormethyl, Methoxymethyl oder Trifluormethoxymethyl steht und R¹ für (C₁-C₆-Alkyl)carbonyl, (C₁-C₆-Alkoxy)carbonyl, (C₁-C₄-Alkoxy-C₁-C₄-alkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl mit jeweils 1 bis 9 Fluor-, Chlor- und/oder Bromatomen steht.
- R²⁹: steht ganz besonders bevorzugt für Fluor, Chlor, Brom, Hydroxy, Methyl, Methoxy, Cyclopropyl, Trifluormethyl, Difluormethyl, Trifluormethoxy oder Difluormethoxy
mit der Maßgabe,
a) dass R²⁹ nicht für Trifluormethyl, Difluormethyl, Methyl oder Ethyl, wenn R²⁸ für Wasserstoff oder Methyl steht und R¹ und R² gleichzeitig für Wasserstoff stehen,
- R³⁰: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R³⁰: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³⁰: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³⁰: steht insbesondere bevorzugt für Amino, Methylamino, Dimethylamino, Methyl oder Trifluormethyl.
- R³¹: steht bevorzugt für Fluor, Chlor, Brom, Methyl, Ethyl oder C₁-C₂-Halogenalkyl mit 1 bis 5 Fluor, Chlor und/oder Bromatomen.
- R³¹: steht besonders bevorzugt für Fluor, Chlor, Brom, Methyl, Ethyl, Trifluormethyl, Difluormethyl, Difluorchlormethyl oder Trichlormethyl.
- R³¹: steht ganz besonders bevorzugt für Fluor, Chlor, Brom, Methyl, Trifluormethyl, Difluormethyl oder Trichlormethyl.
- R³¹: steht insbesondere bevorzugt für Methyl, Trifluormethyl oder Difluormethyl.
- R³²: steht bevorzugt für Wasserstoff, Fluor, Chlor, Brom, Methyl, Ethyl oder C₁-C₂-Halogenalkyl mit 1 bis 5 Fluor, Chlor und/oder Bromatomen,
mit der Maßgabe, dass R³² nicht für Chlor steht, wenn R¹ und R² gleichzeitig für Wasserstoff stehen.
- R³²: steht besonders bevorzugt für Wasserstoff, Fluor, Chlor, Brom, Methyl oder Trifluormethyl, mit der Maßgabe, dass R³² nicht für Chlor steht, wenn R¹ und R² gleichzeitig für Wasserstoff stehen.
- R³³: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R³³: steht besonders bevorzugt für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl.
- R³³: steht ganz besonders bevorzugt für Fluor, Chlor, Brom, Iod, Methyl, Trifluormethyl, Difluormethyl oder Trichlormethyl.
- R³⁴: steht bevorzugt für Methyl, Ethyl, n-Propyl oder iso-Propyl.
- R³⁴: steht besonders bevorzugt Methyl oder Ethyl.

Hervorgehoben sind Verbindungen der Formel (I), in welcher R¹ für Wasserstoff steht.
Hervorgehoben sind Verbindungen der Formel (I), in welcher R¹ für Formyl steht.
Hervorgehoben sind außerdem Verbindungen der Formel (I), in welcher R¹ für -C(=O)C(=O)R³ steht,
wobei R³ die oben angegebenen Bedeutungen hat.
Hervorgehoben sind Verbindungen der Formel (I), in welcher A für A1 steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein. Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Bevorzugt, besonders bevorzugt oder ganz besonders bevorzugt sind Verbindungen der Formel (I), welche jeweils die unter bevorzugt, besonders bevorzugt oder ganz besonders bevorzugt genannten Substituenten tragen.

### Beschreibung der erimdungsgemäßen Verfahren zum Herstellen der Hexylcarboxanilide der Formel (I) sowie der Zwischenprodukte (wobei nur Verfahren, welche zu Verbindungen der Formel (I) wie in den Ansprüchen führen, des erfindungsgemäß anzusehen sind.)

### Verfahren (a)

Verwendet man 4-Methoxy-2-methyl-1,3-thiazol-5-carbonylchlorid und [2-(1,3-Dimethylbutyl)phenyl]amin als Ausgangsstoffe, so kann das erfindungsgemäße Verfahren (a) durch das folgende Formelschema veranschaulicht werden:

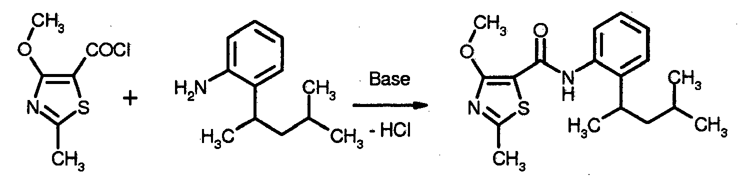

Die zur Durchführung des erfindungsgemäßen Verfahrens (a) als Ausgangsstoffe benötigten Carbonsäure-Derivate sind durch die Formel (II) allgemein definiert. In dieser Formel (II) hat A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden. X¹ steht bevorzugt für Chlor, Brom oder Hydroxy.

Die Carbonsäure-Derivate der Formel (II) sind größtenteils bekannt und/oder lassen sich nach bekannten Verfahren herstellen (vgl. WO 93/11117, EP-A 0 545 099, EP-A 0 589 301 und EP-A 0589313).

### 3-Dichlormethyl-1H-pyrazol-4-carbonsäure-Derivate der Formel (II-a)

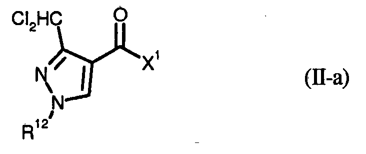

in welcher
- R¹² und X¹: die oben angegebenen Bedeutungen haben,
können erhalten werden, indem man in einem ersten Schritt Ketoacetale der Formel (V) 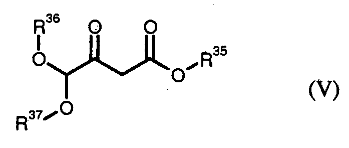 in welcher
- R³⁵: für C₁-C₄-Alkyl, bevorzugt für Methyl, Ethyl, n-, iso-Propyl, n-, sec-, tert-Butyl, steht,
- R³⁶ und R³⁷: jeweils für Methyl oder Ethyl stehen, oder
- R³⁶ und R³⁷: gemeinsam für -(CH₂)₃- oder -CH₂-C(CH₃)₂-CH₂- stehen,
mit Orthoameisensäurealkylestern der Formel (VI)

HC-(OR³⁸)₃ (VI)

in welcher
- R³⁸: für C₁-C₄-Alkyl, bevorzugt für Methyl, Ethyl, n-, iso-Propyl, n-, sec-, tert-Butyl, steht, in Gegenwart eines Anhydrids (z.B. Essigsäureanhydrid) umsetzt
und die so erhaltenen Verbindungen der Formel (VII) 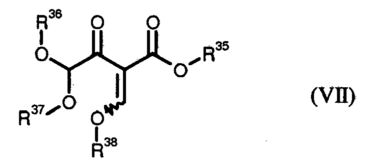 in welcher R³⁵, R³⁶, R³⁷ und R³⁸ die oben angegebenen Bedeutungen haben,
in einem zweiten Schritt mit Hydrazin-Derivaten der Formel (VIII) 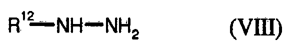 in welcher R¹² die oben angegebenen Bedeutungen hat,
in Gegenwart eines Verdünnungsmittels (z.B. Methanol) umsetzt
und die so erhaltenen Pyrazol-Derivate der Formel (IX) 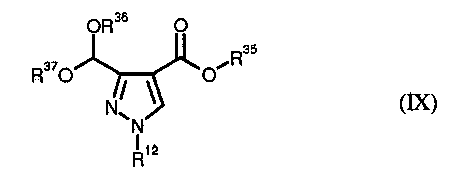 in welcher R¹², R³⁵ R³⁶ und R³⁷ die oben angegebenen Bedeutungen haben,
in einem dritten Schritt in Gegenwart einer Säure (z.B. Salzsäure) und in Gegenwart eines Verdünnungsmittels (z.B. Dioxan) umsetzt und die so erhaltenen 3-Formyl-1H-pyrazol-4-carbonsäureester der Formel (X) 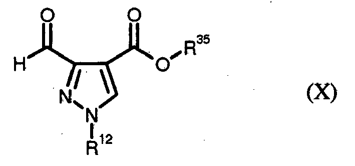 in welcher R¹² und R³⁵ die oben angegebenen Bedeutungen haben,
entweder
a) in einem vierten Schritt in Gegenwart einer Base (z.B. Lithiumhydroxid) und in Gegenwart eines Verdünnungsmittels (z.B. Tetrahydrofuran) verseift und die so erhaltenen 3-Formyl-1H-pyrazol-4-carbonsäuren der Formel (XI) 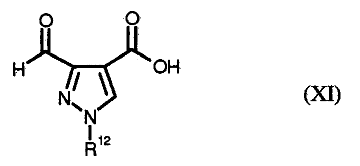 in welcher R¹² die oben angegebenen Bedeutungen hat,
   anschließend mit einem Chlorierungsmittel (z.B. Phosphorpentachlorid) in Gegenwart eines Verdünnungsmittels (z.B. Dichlormethan) umsetzt,
   oder
b) in einem vierten Schritt mit einem Chlorierungsmittel (z.B. Phosphorpentachlorid) in Gegenwart eines Verdünnungsmittels (z.B. Dichlormethan) umsetzt
   und die so erhaltenen 3-Dichlormethyl-1H-pyrazol-4-carbonsäureester der Formel (XII) 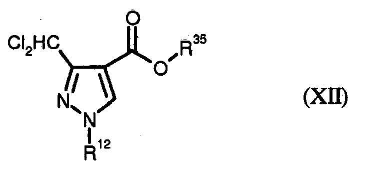 in welcher R¹² und R³⁵ die oben angegebenen Bedeutungen haben, anschließend in Gegenwart einer Base (z.B. Lithiumhydroxid) und in Gegenwart eines Verdünnungsmittels (z.B. Tetrahydrofuran) verseift.

Die zur Durchführung des erfindungsgemäßen Verfahrens (a) als Ausgangsstoffe weiterhin benötigten Anilin-Derivate sind durch die Formel (III) allgemein definiert. In dieser Formel (III) haben R¹ und R² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Die Anilin-Derivate der Formel (III) sind bekannt und/oder lassen sich nach bekannten Verfahren erhalten (vgl. EP-A 0 824 099, WO 02/059086, WO 03/010149). Anilin-Derivate der Formel (III), in welcher R¹ nicht für Wasserstoff steht, lassen sich z.B. herstellen, indem man

### Anilin-Derivate der Formel (III-a)

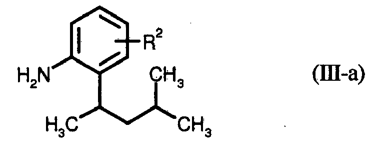

in welcher R² die oben angegebenen Bedeutungen hat,
mit Halogeniden der Formel (IV) 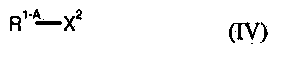 in welcher R^{1-A} die oben angegebenen Bedeutungen hat,
in Gegenwart einer Base und in Gegenwart eines Verdünnungsmittels umsetzt. [Die Reaktionsbedingungen des Verfahrens (b) gelten entsprechend.]

### Verfahren (b)

Verwendet man 1,3,5-Trimethyl-N-[2-(1,3-dimethylbutyl)phenyl]-1H-pyrazol-4-carboxamid und Ethyl-chlor(oxo)acetat als Ausgangsstoffe, so kann der Verlauf des erfindungsgemäßen Verfahrens (b) durch das folgende Formelschema veranschaulicht werden: 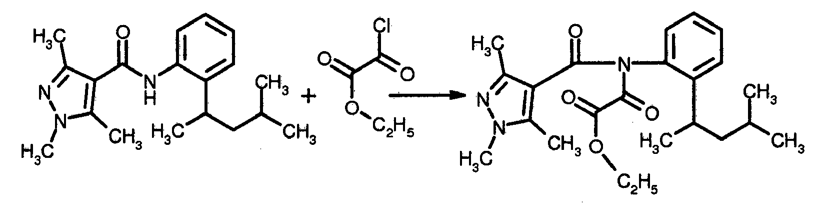 Die zur Durchführung des erfindungsgemäßen Verfahrens (b) als Ausgangsstoffe benötigten Hexylcarboxanilide sind durch die Formel (I-a) allgemein definiert. In dieser Formel (I-a) haben R² und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Hexylcarboxanilide der Formel (I-a) sind ebenfalls erfindungsgemäße Verbindungen und Gegenstand dieser Anmeldung. Sie können nach dem erfindungsgemäßen Verfahren (a) erhalten werden (mit R¹ = Wasserstoff).

Die zur Durchführung des erfindungsgemäßen Verfahrens (b) als Ausgangsstoffe weiterhin benötigten Halogenide sind durch die Formel (IV) allgemein definiert.
- R^{1-A}: steht bevorzugt für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R³, -CONR⁴R⁵ oder -CH₂NR⁶R⁷.
- R^{1-A}: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R³, -CONR⁴R⁵ oder -CH₂NR⁶R⁷.
- R^{1-A}: steht ganz besonders bevorzugt für Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- X²: steht bevorzugt für Chlor oder Brom.

### Halogenide der Formel (IV) sind bekannt.

### Reaktionsbedingungen

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldümidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens (a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro mol des Carbonsäure-Derivates der Formel (II) im Allgemeinen 0,2 bis 5 mol, vorzugsweise 0,5 bis 2 mol an Anilin-Derivat der Formel (III) ein.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (b) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des Hexylcarboxanilids der Formel (I-a) im Allgemeinen 0,2 bis 5 Mol, vorzugsweise 0,5 bis 2 Mol an Halogenid der Formel (IV) ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides,
Rhizoctonia-Arten, wie beispielsweise Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosinesodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]-ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-Cyhalothrin, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

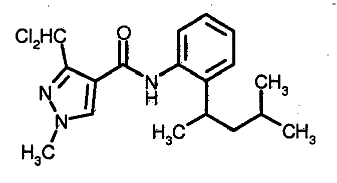

Zu einer Lösung bestehend aus 250.2 mg (1.1 mmol) 3-Dichlormethyl-1-methyl-1H-pyrazol-4-carbonylchlorid und 161.9 mg (1.6 mmol) Triethylamin in 10 ml Tetrahydrofuran werden 177.3 mg (1.0 mmol) 2-(1,3-Dimethyl-butyl)-phenylamin gegeben. Die Reaktionsmischung wird 16 h bei 60°C gerührt, über Silicagel filtriert und im Vakuum aufkonzentriert.

Säulenchromatographie (Cyclohexan/Essigsäureethylester 3:1) liefert 257.6 mg (70 % der Theorie) an 3-(Dichlormethyl)-1-methyl-N-[2-(1,3-dimethylbutyl)phenyl]-1H-pyrazol-4-carboxamid [logP (pH 2.3) = 3.74].

### Beispiel 2

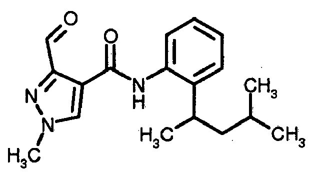

Zu einer Suspension bestehend aus 150.0 mg (0.97 mmol) 3-Formyl-1-methyl-1H-pyrazol-4-carbonsäure in 7 ml Dichlormethan werden 135.9 mg (1.07 mmol) Oxalsäuredichlorid und einige Tropfen Dimethylformamid gegeben. Nach 2 h bei Raumtemperatur wird eine Lösung bestehend aus 172.5 mg (0.97 mmol) 2-(1,3-Dimethyl-butyl)-phenylamin in 7 ml Dichlormethan und 128.0 mg (1.27 mmol) Triethylamin zugetropft. Nach 16 h bei Raumtemperatur wird mit 7 ml 2N Salzsäure versetzt, die organische Phase abgetrennt, über Magnesiumsulfat getrocknet und über Kieselgel filtriert.

Aufkonzentration im Vakuum liefert 273.6 mg (90 % der Theorie) an N-[2-(1,3-Dimethylbutyl)phenyl]-3-formyl-1-methyl-1H-pyrazol-4-carboxamid [log P (pH 2.3) = 3.64].

### Beispiel 3

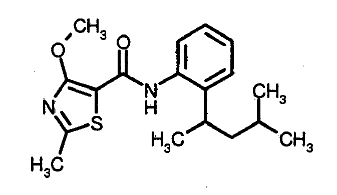

350 mg (1.83 mmol) 4-Methoxy-2-methyl-1,3-thiazol-5-carbonylchlorid und 324 mg (1.83 mmol) an [2-(1,3-Dimethylbutyl)phenyl]amin wurden in 40 ml Acetonitril 20 h bei Raumtemperatur und 8 h bei 50 °C im abgeschlossenen Gefäß unter Argon gerührt. Anschließend wurde mit jeweils 20 ml Wasser und 40 ml Essigsäureethylester versetzt, die organische Phase abgetrennt, mit 30 ml gesättigter Ammoniumchlorid-Lösung und Wasser gewaschen, über Natriumsulfat getrocknet und eingeengt.

Nach säulenchromatographischer Reinigung über Kieselgel 60 (Petrolether/Essigsäureethylester 5:1 → Essigsäureethylester) wurden 420 mg an N-[2-(1,3-Dimethylbutyl)phenyl]-4-methoxy-2-methyl-1,3-thiazol-5-carboxamid erhalten [logP (pH 2.3) = 4.45].

### Beispiel 4

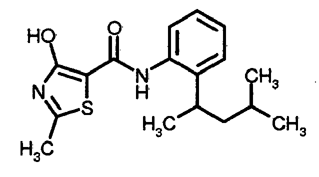

170 mg (0.38 mmol) N-[2-(1,3-Dimethylbutyl)phenyl]-4-methoxy-2-methyl-1,3-thiazol-5-carboxamid (Beispiel 3) und 60 mg wasserfreies Aluminiumchlorid wurden in 8 ml 1,2-Dichlorethan 24 h bei 40-50°C gerührt. Anschließend wurde mit 10 ml Wasser versetzt, die organische Phase abgetrennt, die Wasserphase noch zweimal mit je 30 ml Dichlormethan extrahiert. Die vereinigten organischen Phasen wurden über Natriumsulfat getrocknet und eingeengt.

Nach säulenchromatographischer Reinigung über Kieselgel 60 (Dichlormethan/Diethylether 5:1) wurden 60 mg an N-[2-(1,3-Dimethylbutyl)phenyl]-4-hydroxy-2-methyl-1,3-thiazol-5-carboxamid erhalten [logP (pH 2.3) = 2.68].

Analog den Beispielen 1 bis 4, sowie entsprechend den Angaben in den allgemeinen Verfahrensbeschreibungen, werden die in der nachstehenden Tabelle 1 genannten Verbindungen der Formel (I) erhalten.

**Tabelle 1**

| | | | | | | | | | | |
|---|---|---|---|---|---|---|---|---|---|---|
| 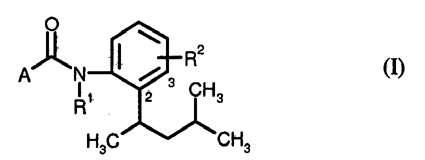 | | | | | | | | | | |

| **Bsp.** | **R¹** | **R²** | **A** | **logP** | | **Bsp.** | **R¹** | **R²** | **A** | **logP** |
|---|---|---|---|---|---|---|---|---|---|---|
| 5 | H | H | 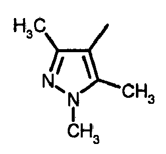 | 2.99 | | 6 | H | H | 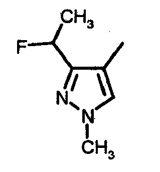 | 3.34 |
| 7 | H | H | 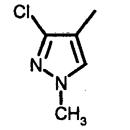 | 3.49 | | 8 | H | H | 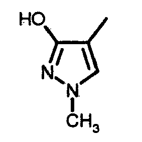 | 2.46 |
| 9 | H | H | 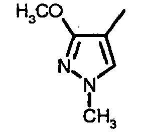 | 3.48 | | 10 | H | H | 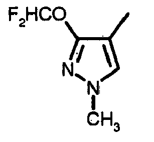 | 3.91 |
| 11 | H | H | 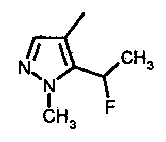 | 3.51 | | 12 | H | 4-F | 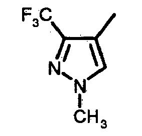 | 3.60 |
| 13 | H | 4-F | 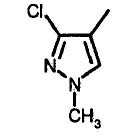 | 3.49 | | 14 | H | 4-F | 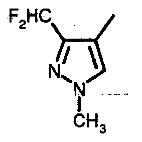 | 3.35 |
| 15 | H | H | 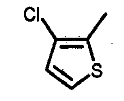 | 4.63 | | 16 | H | H | 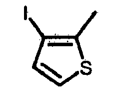 | 4.51 |
| 17 | H | 4-F | 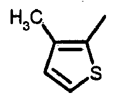 | 4.11 | | 18 | H | H | 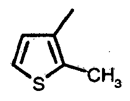 | 4.09 |
| 19 | H | 4-F | 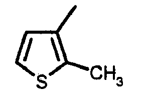 | 4.08 | | 20 | H | 4-F | 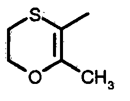 | 3.99 |
| 21 | H | H | 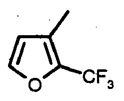 | 4.10 | | 22 | H | 4-F | 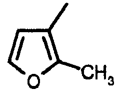 | 3.81 |
| 23 | H | H | 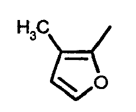 | 4.20 | | 24 | H | H | 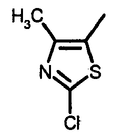 | 4.21 |
| 25 | H | H | 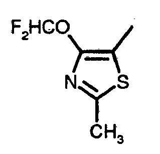 | 4.75 | | 26 | H | H | 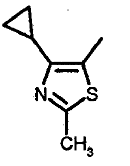 | 4.17 |
| 27 | H | 4-F | 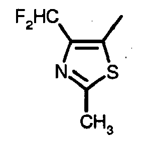 | 3.72 | | 28 | H | 4-F | 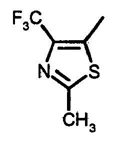 | 4.04 |
| 29 | H | H | 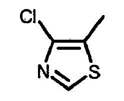 | 3.86 | | 30 | H | H | 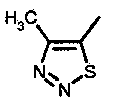 | 3.54 |
| 31 | H | H | 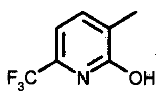 | 3.74 | | 32 | H | H | 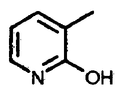 | 2.80 |
| 33 | H | H | 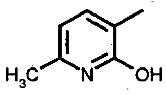 | 2.94 | | 34 | H | H | 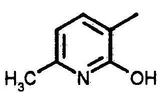 | 3.35 |
| 35 | H | H | 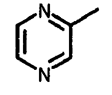 | 3.68 | | 36 | H | 4-F | 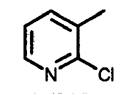 | 3.35 |
| 37 | H | H | 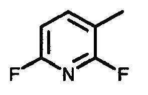 | Sdp. 76-81 °C | | 38 | H | H | 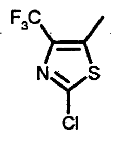 | 4.54 |

### Herstellung von Ausgangsstoffen der Formel (II)

### Beispiel (II-1)

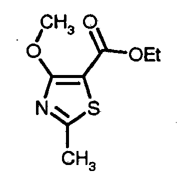

200 mg (1.07 mmol) 4-Hydroxy-2-methyl-1,3-thiazol-5-carbonsäureethylester und 100 mg Kieselgel wurden in 10 ml Dichlormethan vorgelegt, 0.9 ml (1.3 mmol) Trimethylsilyldiazomethan (2N in Hexan) zugespritzt und 3 Tage bei Raumtemperatur gerührt. Anschließend wurde mit 1 ml Methanol und dann 5 ml Wasser versetzt. Die organische Phase wurde abgetrennt und die Wasserphase noch zweimal mit Dichlormethan extrahiert. Die vereinigten organischen Phasen wurden noch zweimal mit je 20 ml Wasser gewaschen, über Natriumsulfat getrocknet und eingeengt. Man erhielt 196 mg (91 % der Theorie) an 4-Methoxy-2-methyl-thiazol-5-carbonsäureethylester [logP (pH 2.3) = 1.90].

### Beispiel (II-2)

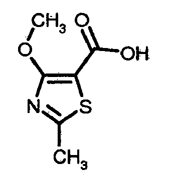

210 mg (1.0 mmol) 4-Methoxy-2-methyl-thiazol-5-carbonsäureethylester wurde in 5 ml Ethanol vorgelegt und 123 mg (2.2 mmol) Kaliumhydroxid, gelöst in 1 ml Wasser, zugegeben. Nach 4 h Rühren bei Raumtemperatur und 30 h unter Rückfluss wurde eingeengt. Der Rückstand wurde in 30 ml Wasser aufgenommen und zweimal mit 30 ml Diethylether extrahiert. Die wässrige Phase wurde mit verdünnter Salzsäure angesäuert und erneut dreimal mit je 30 ml Essigsäureethylester extrahiert. Die vereinigten org. Extrakte wurden über Natriumsulfat getrocknet und eingeengt. Man erhielt 185 mg (quantitativ) an 4-Methoxy-2-methyl-thiazol-5-carbonsäure [logP (pH 2.3) = 0.77].

### Beispiel (II-3)

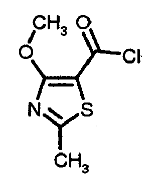

3.35 g (19.3 mmol) 4-Methoxy-2-methyl-1,3-thiazol-5-carbonsäure und 11.5 g Thionylchlorid wurden in 30 ml Toluol 3 h bei 85°C gerührt. Nach dem Einengen wurde der Rückstand dreimal mit je 10 ml Dichlormethan versetzt und eingeengt. Man erhielt 3.3 g (89 % der Theorie) an 4-Methoxy-2-methyl-thiazol-5-carbonylchlorid [Nachweis über den Methylester: logP (pH 2.3) = 1.45].

### Beispiel (II-4)

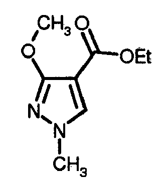

2.60 g (15.3 mmol) 3-Hydroxy-1-methyl-1H-pyrazol-4-carbonsäureethylester und 0.43 g Kieselgel wurden in 140 ml Dichlormethan vorgelegt, 12.7 ml (18.3 mmol) Trimethylsilyldiazomethan (2N in Hexan) zugespritzt und 2 Tage bei Raumtemperatur gerührt. Nach erneutem Zusetzen von 2 ml Trimethylsilyldiazomethan wurde weitere 24 h bei Raumtemperatur gerührt. Anschließend wurde mit 1 ml Methanol, dann mit 100 ml Wasser versetzt, die organische Phase abgetrennt und die Wasserphase noch zweimal mit je 40 ml Dichlormethan extrahiert. Die vereinigten organischen Phasen wurden über Natriumsulfat getrocknet und eingeengt. Nach säulenchromatographischer Reinigung über Kieselgel 60 mit Essigsäureethylester/Hexan 3:1 wurden 1.4 g (50 % der Theorie) an 3-Methoxy-1-methyl-1H-pyrazol-4-carbonsäureethylester erhalten [logP (pH 2.3) = 1.14].

### Beispiel (II-5)

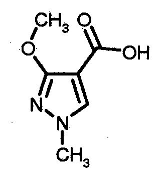

4.6 g (27.0 mmol) 3-Methoxy-1-methyl-1H-pyrazol-4-carbonsäuremethylester wurde in 40 ml Ethanol vorgelegt und 3.19 g (56.8 mmol) Kaliumhydroxid, gelöst in 10 ml Wasser, zugegeben. Nach 18 h Rühren bei Raumtemperatur und 4 h bei 40°C wurde eingeengt, der Rückstand mit 50 ml Wasser aufgenommen, zweimal mit je 30 ml Diethylether extrahiert. Die wässrige Phase wurde mit Salzsäure angesäuert und erneut dreimal mit je 30 ml Essigsäureethylester extrahiert. Die vereinigten organischen Extrakte wurden über Natriumsulfat getrocknet und eingeengt. Man erhielt 3.9 g (92 % der Theorie) an 3-Methoxy-1-methyl-1H-pyrazol-4-carbonsäure.

### Beispiel (II-6)

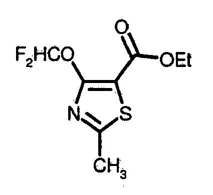

5.0 g (26.7 mmol) 4-Hydroxy-2-methyl-1,3-thiazol-5-carbonsäureethylester und 7.4 g Kaliumcarbonat wurden in 30 ml Dimethylformamid vorgelegt und auf 100°C erwärmt. 2.3 g (26.7 mmol) Frigen wurde über 3 h eingeleitet. Nach dem Abkühlen wurde eingeengt und der Rückstand mit 100 ml Wasser/Essigsäureethylester versetzt, die organische Phase abgetrennt und noch dreimal mit Wasser gewaschen. Die organische Phase wurde über Natriumsulfat getrocknet und eingeengt. Man erhielt 5.6 g (88 % der Theorie) an 4-(Difluormethoxy)-2-methyl-1,3-thiazol-5-carbonsäureethylester [logP (pH 2.3) = 2.54].

### Beispiel (II-7)

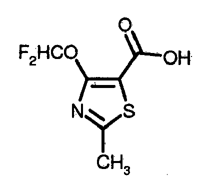

5.5 g (23.2 mmol) 4-(Difluormethoxy)-2-methyl-1,3-thiazol-5-carbonsäureethylester wurde in 40 ml Ethanol vorgelegt und 1.4 g (25.5 mmol) Kaliumhydroxid, gelöst in 10 ml Wasser, zugegeben. Nach 16 h Rühren bei Raumtemperatur wurde eingeengt, der Rückstand in 80 ml Wasser aufgenommen, zweimal mit je 40 ml Essigsäureethylester extrahiert, die wässrige Phase mit Salzsäure angesäuert und erneut dreimal mit je 60 ml Essigsäureethylester extrahiert. Die vereinigten organischen Extrakte wurden über Natriumsulfat getrocknet und eingeengt.

Man erhielt 3.9 g (80 % der Theorie) an 4-(Difluormethoxy)-2-methyl-1,3-thiazol-5-carbonsäure [logP (pH 2.3) = 1.29].

### Beispiel (II-8)

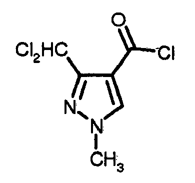

300 mg (1.9 mmol) 3-Formyl-1-methyl-1H-pyrazole-4-carbonsäure werden in 60 ml Dichlormethan gelöst und mit 1.0 g (4.9 mmol) Phosphorpentachlorid versetzt. Nach 1.5 h bei Raumtemperatur wird auf Eiswasser gegeben, mit Dichlormethan extrahiert, über Magnesiumsulfat getrocknet, filtriert und im Vakuum aufkonzentriert. Man erhält 384 mg (86 % der Theorie) an 3-Dichlormethyl-1-methyl-1H-pyrazol-4-carbonyl chlorid.

### Herstellung von Ausgangsstoffen der Formel (VII)

### Beispiel (VII-1)

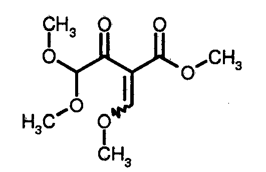

Zu einer Lösung bestehend aus 10.0 g (57 mmol) 4,4-Dimethoxy-3-oxo-buttersäure-methylester in 9.0 g (85 mmol) Orthoameisensäuretrimethylester werden 16.0 ml (170 mol) Essigsäureanhydrid gegeben. Die Reaktionsmischung wird für 16 h unter Rückfluss erhitzt.
Destillation aus der Reaktionsmischung (Siedepunkt 132-135°C, 0.2 bar) liefert 7.0 g (56 % der Theorie) an 4,4-Dimethoxy-2-methoxymethylen-3-oxo-buttersäuremethylester.

### Herstellung von Ausgangsstoffen der Formel (IX)

### Beispiel (IX-1)

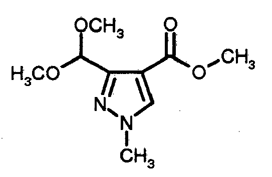

Bei -5°C wird eine Lösung bestehend aus 2.0 ml (38 mmol) Methylhydrazin in 340 ml Methanol langsam zu 7.5 g 4,4-Dimethoxy-2-methoxymethylen-3-oxo-buttersäuremethylester getropft. Nach beendeter Zugabe wird die Reaktionsmischung für 16 h bei Raumtemperatur gerührt und im Vakuum aufkonzentriert.
Säulenchromatographie (Laufmittelgradient Cyclohexan/Essigsäureethylester) liefert 6.5 g (77 % der Theorie) an 3-Dimethoxymethyl-1-methyl-1H-pyrazol-4-carbonsäuremethylester.

### Herstellung von Ausgangsstoffen der Formel (X)

### Beispiel (X-1)

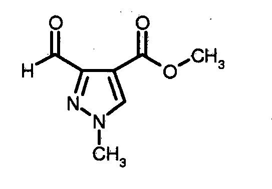

Eine Lösung aus 2.1 g (10 mmol) 3-Dimethoxymethyl-1-methyl-1H-pyrazol-4-carbonsäuremethylester in 20 ml Dioxan wird mit 10 ml konzentrierter Salzsäure versetzt und für 16 h bei Raumtemperatur gerührt. Zur Aufarbeitung wird im Vakuum aufkonzentriert, der Rückstand mit 200 ml Methylenchlorid aufgenommen und mit 50 ml Wasser gewaschen. Die organische Phase wird über Magnesiumsulfat getrocknet, filtriert und aufkonzentriert.
Man erhält 1.6 g (94 % der Theorie) an 3-Formyl-1-methyl-1H-pyrazole-4-carbonsäuremethylester [logP (pH 2.3) = 0.46].

### Herstellung von Ausgangsstoffen der Formel (XI)

### Beispiel (XI-1)

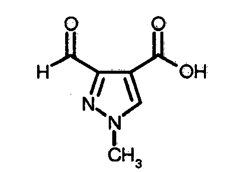

6.0 g (35.68 mmol) 3-Formyl-1-methyl-1H-pyrazol-4-carbonsäuremethylester werden in 180 ml Tetrahydrofuran und 90 ml Wasser gelöst und mit 0.94 g (39.25 mmol) Lithiumhydroxid versetzt. Die Reaktionsmischung wird für 16 h bei Raumtemperatur gerührt, das organische Lösungsmittel im Vakuum entfernt, die verbleibende wässrige Phase mit verdünnter Salzsäure angesäuert, dreimal mit je 100 ml Essigsäureethylester extrahiert. Die organischen Phasen werden über Magnesiumsulfat getrocknet, filtriert und aufkonzentriert. Man erhält so 4.28 g (78 % der Theorie) an 3-Formyl-1-methyl-1H-pyrazole-4-carbonsäure mit dem logP (pH = 2.3) = -0.19.

### Herstellung von Ausgangsstoffen der Formel (XII)

### Beispiel (XII-1)

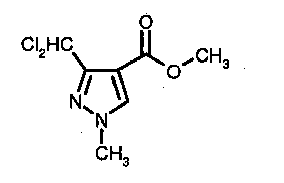

46.1 mg (0.27 mmol) 3-Formyl-1-methyl-1H-pyrazol-4-carbonsäuremethylester werden in 10 ml Dichlormethan gelöst und mit 142.9 mg (0.67 mmol) Phosphorpentachlorid versetzt. Die Reaktionsmischung wird für 1.5 h bei Raumtemperatur gerührt, auf Wasser gegeben, mit Diethylether extrahiert, über Magnesiumsulfat getrocknet und im Vakuum konzentriert. Man erhält so 53.0 mg (86 % der Theorie) an 3-(Dichlormethyl)-1-methyl-1H-pyrazol-4-carbonsäuremethylester mit dem logP (pH 2.3) = 1.80.

Dieser Methylester kann auf übliche Weise verseift werden. Man erhält die 3-(Dichlormethyl)-1-methyl-1H-pyrazol-4-carbonsäure, welche entweder direkt mit Verbindungen der Formel (III) gekuppelt wird oder zuvor in das Säurechlorid überführt wird.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Podosphaera-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Podosphaera-Test (Apfel) / protektiv** | | |
|---|---|---|
| Wirkstoff Erfindungsgemäß | Aufwandmenge an Wirkstoff in g/ha | Wirkungsgrad in % |
| 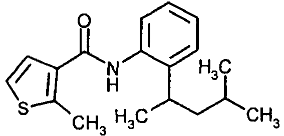 | 100 | 100 |
| 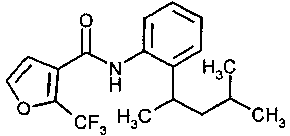 | 100 | 98 |
| 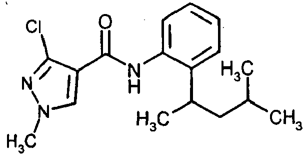 | 100 | 100 |
| 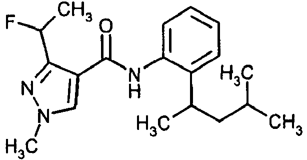 | 100 | 100 |

### Beispiel B

### Venturi - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | |
|---|---|---|
| Wirkstoff Erfindungsgemäß | Aufwandmenge an Wirkstoff in g/ha | Wirkungsgrad in % |
| 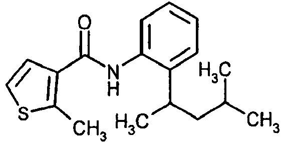 | 100 | 100 |
| 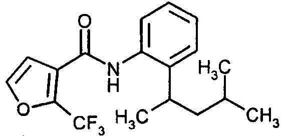 | 100 | 96 |
| 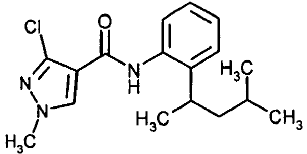 | 100 | 100 |
| 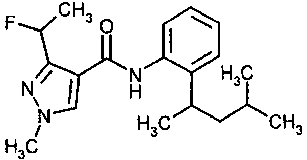 | 100 | 100 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | |
|---|---|---|
| Wirkstoff Erfindungsgemäß | Aufwandmenge an Wirkstoff in g/ha | Wirkungsgrad in % |
| 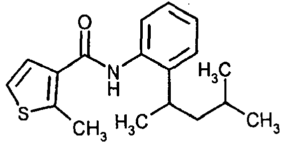 | 100 | 97 |
| 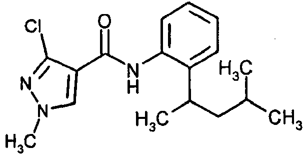 | 100 | 100 |
| 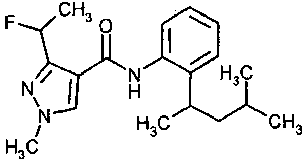 | 500 | 99 |

### Beispiel D

### Puccinia-Test (Weizen) / protektiv

| | | |
|---|---|---|
| Lösungsmittel: | 50 | Gewichtsteile N,N-Dimethylacetamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabihe.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 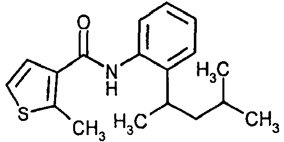 | 500 | 100 |
| 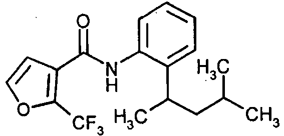 | 500 | 100 |
| 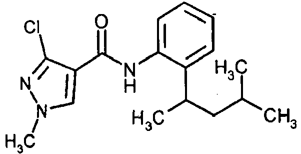 | 500 | 100 |

### Beispiel E

### Sphaerotheca-Test (Gurke) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge, 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Sphaerotheca-Test (Gurke) / protektiv** | | |
|---|---|---|
| Wirkstoff Erfindungsgemäß | Aufwandmenge an Wirkstoff in g/ha | Wirkungsgrad in % |
| 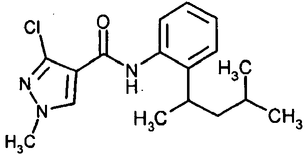 | 750 | 100 |

## Patentansprüche

1. 1,3-Dimethylbutylcarboxanilide der Formel (I) 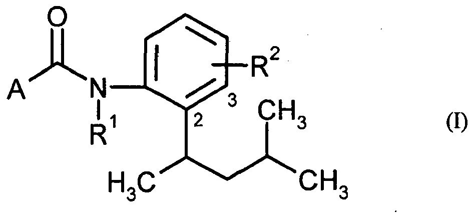 in welcher
R¹ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; oder -C(=O)C(=O)R³, -CONR⁴R³ oder -CH₂NR⁶R⁷ steht,
R² für Wasserstoff steht,
R³ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R⁴ und R⁵ unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁴ und R⁵ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁸ enthalten kann,
R⁶ und R⁷ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R⁶ und R⁷ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁸ enthalten kann,
R⁸ für Wasserstoff oder C₁-C₆-Alkyl steht,
A für den Rest der Formel (A1) 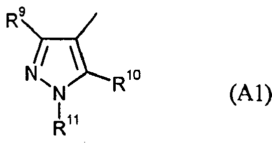 steht, in welcher
R⁹ für Fluor, Chlor, Brom, oder C₂-Halogenalkyl mit 1 bis 5 Halogenatomen steht,
R¹⁰ für Wasserstoff, Chlor, Brom, Iod, Cyano, C₁-C₄-Alkyl, C₁-C₄-Alkoxy, C₁-C₄-Alkylthio oder C₁-C₄-Halogenalkyl mit 1 bis 5 Halogenatomen steht,
R¹¹ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht,
oder
A für den Rest der Formel (A2) 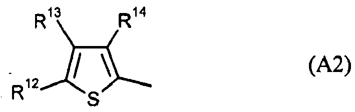 steht, in welcher
R¹² und R¹³ unabhängig voneinander für Wasserstoff, Halogen, C₁-C₄-Alkyl oder C₁-C₄-Halogenalkyl mit jeweils 1 bis 5 Halogenatomen stehen und
R¹⁴ für Halogen, Cyano oder C₁-C₄-Alkyl, oder C₁-C₄-Halogenalkyl oder C₁-C₄-Halogenalkoxy mit jeweils 1 bis 5 Halogenatomen steht,
mit der Maßgabe, dass R¹⁴ nicht für Methyl steht, wenn R¹² und R¹³ für Wasserstoff oder Methyl stehen und R¹ und R² gleichzeitig für Wasserstoff stehen,
oder
A für den Rest der Formel (A3) 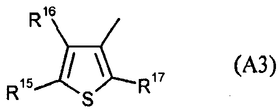 steht, in welcher
R¹⁵ und R¹⁶ unabhängig voneinander für Wasserstoff, Halogen, C₁-C₄-Alkyl oder C₁-C₄-Halogenalkyl mit 1 bis 5 Halogenatomen stehen und
R¹⁷ für Wasserstoff, C₁-C₄-Alkyl oder C₁-C₄-Halogenalkyl mit 1 bis 5 Halogenatomen steht,
oder
A für den Rest der Formel (A4) 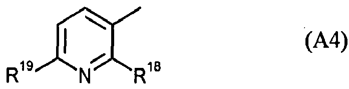 steht, in welcher
R¹⁸ für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R¹⁹ für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulfinyl oder C₁-C₄-Alkylsulfonyl steht,
mit der Maßgabe,
a) dass R¹⁸ nicht für Trifluormethyl, Methyl, Chlor oder Methylthio steht, wenn R¹⁹ für Wasserstoff steht und R¹ und R² gleichzeitig für Wasserstoff stehen,
oder
A für den Rest der Formel (A5) 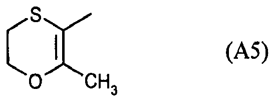 steht,
mit der Maßgabe, dass R¹ und R² nicht gleichzeitig für Wasserstoff stehen, wenn A für A5 steht,
oder
A für den Rest der Formel (A6) 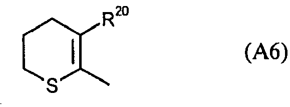 steht, in welcher
R²⁰ für C₁-C₄-Alkyl oder C₁-C₄-Halogenalkyl mit 1 bis 5 Halogenatomen steht,
oder
A für den Rest der Formel (A7) 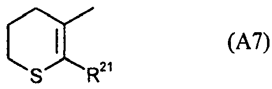 steht, in welcher
R²¹ für C₁-C₄-Alkyl oder C₁-C₄-Halogenalkyl mit 1 bis 5 Halogenatomen steht,
oder
A für den Rest der Formel (A8) 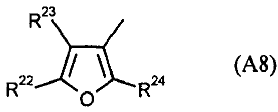 steht, in welcher
R²² und R²³ unabhängig voneinander für Wasserstoff, Halogen, Amino, C₁-C₄-Alkyl oder C₁-C₄-Halogenalkyl mit 1 bis 5 Halogenatomen steht und
R²⁴ für Wasserstoff, C₁-C₄-Alkyl oder C₁-C₄-Halogenalkyl mit 1 to 5 Halogenatomen steht,*
mit der Maßgabe, dass R²⁴ nicht für Methyl steht, wenn R²² und R²³ für Wasserstoff oder Methyl stehen und R¹ und R² gleichzeitig für Wasserstoff stehen,
oder
A für den Rest der Formel (A9) 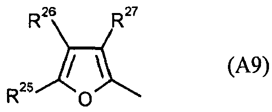 steht, in welcher
R²⁵ und R²⁶ unabhängig voneinander für Wasserstoff, Halogen, Amino, Nitro, C₁-C₄-Alkyl oder C₁-C₄-Halogenalkyl mit 1 bis 5 Halogenatomen stehen und
R²⁷ für Halogen, C₁-C₄-Alkyl oder C₁-C₄-Halogenalkyl mit 1 bis 5 Halogenatomen steht,
oder
A für den Rest der Formel (A11) 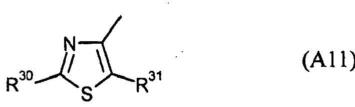 steht, in welcher
R³⁰ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht und
R³¹ für Halogen, C₁-C₄-Alkyl oder C₁-C₄-Halogenalkyl mit 1 bis 5 Halogenatomen steht,
oder
A für den Rest der Formel (A12) 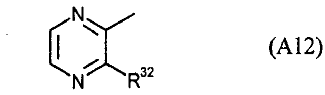 steht, in welcher
R³² für Wasserstoff, Halogen, C₁-C₄-Alkyl oder C₁-C₄-Halogenalkyl mit 1 bis 5 Halogenatomen steht,
mit der Maßgabe, dass R³² nicht für Chlor steht, wenn R¹ und R² gleichzeitig für Wasserstoff stehen,
oder
A für den Rest der Formel (A13) 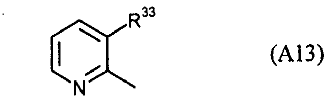 steht, in welcher
R³³ für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
A für den Rest der Formel (A14) 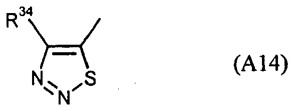 steht, in welcher
R³⁴ für C₁-C₄-Alkyl steht.

2. 1,3-Dimethylbutylcarboxanilide der Formel (I) gemäß Anspruch 1, in welcher
R¹ für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- u nd/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; oder -C(=O)C(=O)R³, -CONR⁴R⁵ oder -CH₂NR⁶R⁷ steht,
R² für Wasserstoff steht,
R³ für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen steht,
R⁴ und R⁵ unabhängig voneinander für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen stehen,
R⁴ und R⁵ außerdem gemeinsam mit dem Stickstoffatom, an das sie gebunden sind, einen gegebenenfalls einfach bis vierfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR⁸ enthalten kann,
R⁶ und R⁷ unabhängig voneinander für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen stehen,
R⁶ und R⁷ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR⁸ enthalten kann,
R⁸ für Wasserstoff oder C₁-C₄-Alkyl steht,
A für den Rest der Formel (A1) 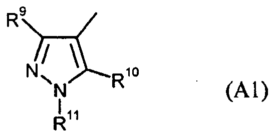 steht, in welcher
R⁹ für Fluor, Chlor, Brom oder C₂-Halogenalkyl mit 1 bis 5 Fluor, Chlor und/oder Bromatomen steht,
R¹⁰ für Wasserstoff, Chlor, Brom, Iod, Methyl, Ethyl, Methoxy, Ethoxy, Methylthio, Ethylthio oder C₁-C₂-Halogenalkyl mit 1 bis 5 Halogenatomen steht,
R¹¹ für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl steht,
oder
A für den Rest der Formel (A2) 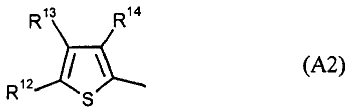 steht, in welcher
R¹² und R¹³ unabhängig voneinander für Wasserstoff, Fluor, Chlor, Brom, Methyl, Ethyl oder C₁-C₂-Halogenalkyl mit 1 bis 5 Fluor, Chlor und/oder Bromatomen stehen,
R¹⁴ für Fluor, Chlor, Brom, Iod, Cyano, Methyl, Ethyl, C₁-C₂-Halogenalkyl oder C₁-C₂-Halogenalkoxy mit jeweils 1 bis 5 Fluor, Chlor und/oder Bromatomen steht,
mit der Maßgabe, dass R¹⁴ nicht für Methyl steht, wenn R¹² und R¹³ für Wasserstoff oder Methyl stehen und R¹ und R² gleichzeitig für Wasserstoff stehen,
oder
A für den Rest der Formel (A3) 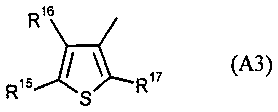 steht, in welcher
R¹⁵ und R¹⁶ unabhängig voneinander für Wasserstoff, Fluor, Chlor, Brom, Methyl, Ethyl oder C₁-C₂-Halogenalkyl mit 1 bis 5 Fluor, Chlor und/oder Bromatomen steht,
R¹⁷ für Wasserstoff, Methyl, Ethyl oder C₁-C₂-Halogenalkyl mit 1 bis 5 Fluor, Chlor und/oder Bromatomen steht,
oder
A für den Rest der Formel (A4) 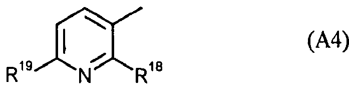 steht, in welcher
R¹⁸ für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen steht,
R¹⁹ für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulfinyl oder C₁-C₂-Alkylsulfonyl steht,
mit der Maßgabe,
a) dass R¹⁸ nicht für Trifluormethyl, Methyl, Chlor oder Methylthio steht, wenn R¹⁹ für Wasserstoff steht,
oder
A für den Rest der Formel (A5) 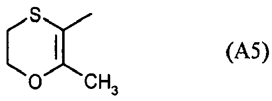 steht,mit der Maßgabe, dass R¹ und R² nicht gleichzeitig für Wasserstoff stehen, wenn A für A5 steht,
oder
A für den Rest der Formel (A8) 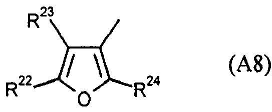 steht, in welcher
R²² und R²³ unabhängig voneinander für Wasserstoff, Fluor, Chlor, Brom, Amino, Methyl, Ethyl oder C₁-C₂-Halogenalkyl mit 1 bis 5 Fluor, Chlor und/oder Bromatomen stehen,
R²⁴ für Wasserstoff, Methyl, Ethyl oder C₁-C₂-Halogenalkyl mit 1 bis 5 Fluor, Chlor und/oder Bromatomen steht,
oder
A für den Rest der Formel (A9) 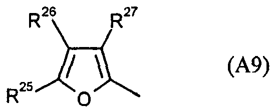 steht, in welcher
R²⁵ und R²⁶ unabhängig voneinander für Wasserstoff, Fluor, Chlor, Brom, Amino, Nitro, Methyl, Ethyl oder C₁-C₂-Halogenalkyl mit 1 bis 5 Fluor, Chlor und/oder Bromatomen stehen,
R²⁷ für Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A11) 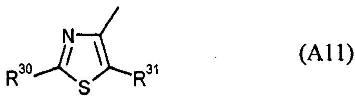 steht, in welcher
R³⁰ für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen steht,
R³¹ für Fluor, Chlor, Brom, Methyl, Ethyl oder C₁-C₂-Halogenalkyl mit 1 bis 5 Fluor, Chlor und/oder Bromatomen steht,
oder
A für den Rest der Formel (A12) 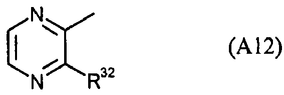 steht, in welcher
R³² für Wasserstoff, Fluor, Chlor, Brom, Methyl, Ethyl oder C₁-C₂-Halogenalkyl mit 1 bis 5 Fluor, Chlor und/oder Bromatomen steht,
mit der Maßgabe, dass R³² nicht für Chlor steht, wenn R¹ und R² gleichzeitig für Wasserstoff stehen,
oder
A für den Rest der Formel (A13) 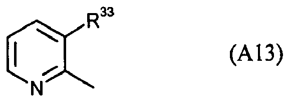 steht, in welcher
R³³ für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen steht.

3. 1,3-Dimethylbutylcarboxanilide der Formel (I) gemäß Anspruch 1 oder 2, in welcher R¹ für Formyl steht.

4. 1,3-Dimethylbutylcarboxanilide der Formel (I) gemäß Anspruch 1 oder 2, in welcher R¹ für -C(=O)C(=O)R³ steht, wobei R³ die in Anspruch 1 oder 2 angegebenen Bedeutungen hat.

5. 1,3-Dimethylbutylcarboxanilide der Formel (I) gemäß Anspruch 1 oder 2, in welcher A für A1 steht.

6. Verfahren zum Herstellen der Verbindungen der Formel (I) gemäß Anspruch 1, **dadurch gekennzeichnet, dass** man
a) Carbonsäure-Derivate der Formel (II) 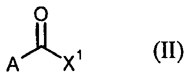 in welcher
A die in Anspruch 1 angegebenen Bedeutungen hat und
X¹ für Halogen oder Hydroxy steht, mit Anilin-Derivaten der Formel (III) 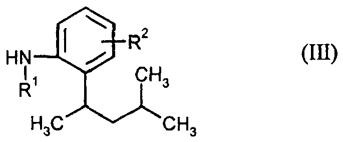 in welcher R¹ und R² die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
oder
b) Hexylcarboxanilide der Formel (I-a) 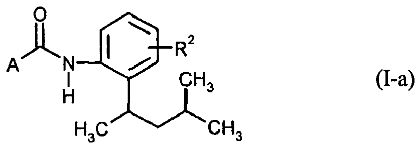 in welcher A und R² die in Anspruch 1 angegebenen Bedeutungen haben mit Halogeniden der Formel (N)
R^{1-A}-X² (IV)
in welcher
X² für Chlor, Brom oder Iod steht,
R¹ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor-und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; ;oder -C(=O)C(=O)R³, CONR⁴R⁵ oder -CH₂NR⁶R⁷ steht, wobei R³, R⁴, R⁵, R⁶ und R⁷ die in Anspruch 1 angegebenen Bedeutungen haben, in Gegenwart einer Base und in Gegenwart eines Verdünnungsmittels umsetzt.

7. Mittel zum Bekämpfen unerwünschter Mikroorganismen, **gekennzeichnet durch** einen Gehalt an mindestens einem 1,3-Dimethylbutylcarboxanilid der Formel (I) gemäß Anspruch 1 neben Streckmitteln und/oder oberflächenaktiven Stoffen.

8. Verwendung von 1,3-Dimethylbutylcarboxaniliden der Formel (I) gemäß Anspruch 1 zum Bekämpfen unerwünschter Mikroorganismen im Pflanzenschutz, im Materialschutz und bei der Behandlung von Saatgut.

9. Verfahren zum Bekämpfen unerwünschter Mikroorganismen im Pflanzenschutz, im Materialschutz und bei der Behandlung von Saatgut, **dadurch gekennzeichnet, dass** man 1,3-Dimethylbutylcarboxanilide der Formel (I) gemäß Anspruch 1 auf die Mikroorganismen und/oder deren Lebensraum ausbringt.

10. Verfahren zum Herstellen von Mitteln zum Bekämpfen unerwünschter Mikroorganismen, **dadurch gekennzeichnet, dass** man 1,3-Dimethylbutylcarboxanilide der Formel (I) gemäß Anspruch 1 mit Streckmitteln und/oder oberflächenaktiven Stoffen vermischt.

## Claims

1. 1,3-Dimethylbutylcarboxanilides of the formula (I) 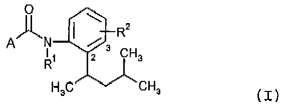 in which
R¹ represents hydrogen, C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C4-alkyl, C₃-C₈-halocycloalkyl having in each case 1 to 9 fluorine, chlorine and/or bromine atoms; formyl, formyl-C₁-C₃-alkyl, (C₁-C₃-alkyl) carbonyl-C₁-C₃-alkyl, (C₁-C₃-alkoxy) carbonyl-C₁-C₃-alkyl; halo-(C₁-C₃-alkyl)carbonyl-C₁-C₃-alkyl, halo- (C₁-C₃-alkoxy)carbonyl-C₁-C₃-alkyl having in each case 1 to 13 fluorine, chlorine and/or bromine atoms; or -C(=O) C(=O)R³, -CONR⁴R⁵ or -CH₂NR⁶R⁷,
R² represents hydrogen,
R³ represents hydrogen, C₁-C₈-alkyl, C₁-C₈-alkoxy, C₁-C₄-alkoxy-C₁-C₄-alkyl, C₃-C₈-cycloalkyl; Cm C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms,
R⁴ and R⁵ independently of one another each represent hydrogen, C₁-C₈-alkyl, C₁-C₄-alkoxy-C₁-C₄-alkyl, C₃-C₈-cycloalkyl, C₁-C₈-haloalkyl, halo-C₁-C₄-alkoxy-C₁-C₄-alkyl, C₃-C₈-halocycloalkyl having in each case 1 to 9 fluorine, chlorine and/or bromine atoms,
R⁴ and R⁵ furthermore together with the nitrogen atom to which they are attached form a saturated heterocycle having 5 to 8 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR⁸,
R⁶ and R⁷ independently of one another represent hydrogen, C₁-C₈-alkyl, C₃-C₈-cycloalkyl; C₁-C₈-haloalkyl, C₃-C₈-halocycloalkyl having in each case 1 to 9 fluorine, chlorine and/or bromine atoms,
R⁶ and R⁷ furthermore together with the nitrogen atom to which they are attached form a saturated heterocycle having 5 to 8 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR⁸,
R⁵ represents hydrogen or C₁-C₆-alkyl,
A represents the radical of the formula (A1) 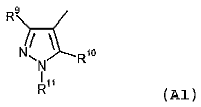 in which
R⁹ represents fluorine, chlorine, bromine, or C₂-haloalkyl having 1 to 5 halogen atoms,
R¹⁰ represents hydrogen, chlorine, bromine, iodine, cyano, C₁-C₄-alkyl, C₁-C₄-alkoxy, C₁-C₄-alkylthio or C₁-C₄-haloalkyl having 1 to 5 halogen atoms,
R¹¹ represents hydrogen, C₁-C₄-alkyl, hydroxyl-C₁-C₄-alkyl, C₂-C₆-alkenyl, C₃-C₆-cycloalkyl, C₁-C₄-alkylthio-C₁-C₄-alkyl, C₁-C₄-alkoxy-C₁-C₄-alkyl, C₁-C₄-haloalkyl, C₁-C₄-haloalkylthio-C₁-C₄-alkyl, C₁-C₄-haloalkoxy-C₁-C₄-alkyl having in each case 1 to 5 halogen atoms, or represents phenyl,
or
A represents the radical of the formula (A2) 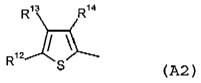 in which
R¹² and R¹³ independently of one another represent hydrogen, halogen, C₁-C₄-alkyl or C₁-C₄-haloalkyl having in each case 1 to 5 halogen atoms and
R¹⁴ represents halogen, cyano or C₁-C₄-alkyl, or C₁-C₄-haloalkyl or C₁-C₄-haloalkoxy having in each case 1 to 5 halogen atoms,
with the proviso that R¹⁴ does not represent methyl if R¹² and R¹³ represent hydrogen or methyl and R¹ and R² simultaneously represent hydrogen,
or
A represents the radical of the formula (A3) 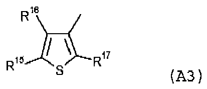 in which
R¹⁵ and R¹⁶ independently of one another represent hydrogen, halogen, C₁-C₄-alkyl or C₁-C₄-haloalkyl having 1 to 5 halogen atoms and
R¹⁷ represents hydrogen, C₁-C₄-alkyl or C₁-C₄-haloalkyl having 1 to 5 halogen atoms,
or
A represents the radical of the formula (A4) 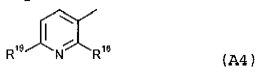 in which
R¹⁸ represents halogen, hydroxyl, cyano, C₁-C₄-alkyl, C₁-C₄-alkoxy, C₁-C₄-alkylthio, C₁-C₄-haloalkyl, C₁-C₄-haloalkylthio or C₁-C₄-haloalkoxy having in each case 1 to 5 halogen atoms,
R¹⁹ represents hydrogen, halogen, cyano, C₁-C₄-alkyl, C₁-C₄-alkoxy, C₁-C₄-alkylthio, C₁-C₄-haloalkyl, C₁-C₄-haloalkoxy having in each case 1 to 5 halogen atoms, C₁-C₄-alkylsulphinyl or C₁-C₄-alkylsulphonyl, with the proviso,
a) hat R¹⁸ does not represent trifluoromethyl, methyl, chlorine or methylthio if R¹⁹ represents hydrogen and R¹ and R² simultaneously represent hydrogen,
or
A represents the radical of the formula (A5) 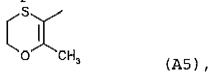 with the proviso, that R¹ and R² do not simultaneously represent hydrogen if A represents A5,
or
A represents the radical of the formula (A6) 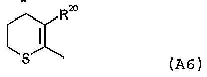 in which
R²⁰ represents C₁-C₄-alkyl or C₁-C₄-haloalkyl having 1 to 5 halogen atoms,
or
A represents the radical of the formula (A7) 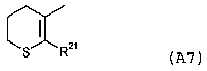 in which
R²¹ represents C₁-C₄-alkyl or C₁-C₄-haloalkyl having 1 to 5 halogen atoms,
or
A represents the radical of the formula (A8) 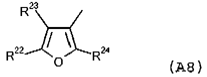 in which
R²² and R²³ independently of one another represent hydrogen, halogen, amino, C₁-C₄-alkyl or C₁-C₄-haloalkyl having 1 to 5 halogen atoms and
R²⁴ represents hydrogen, C₁-C₄-alkyl or C₁-C₄-haloalkyl having 1 to 5 halogen atoms, with the proviso that R²⁴ does not represent methyl if R²² and R²³ represent hydrogen or methyl and R¹ and R² simultaneously represent hydrogen, or
or
A represents the radical of the formula (A9) 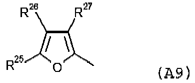 in which
R²⁵ and R²⁶ independently of one another represent hydrogen, halogen, amino, nitro, C₁-C₄-alkyl or C₁-C₄-haloalkyl having 1 to 5 halogen atoms and
R²⁷ represents halogen, C₁-C₄-alkyl or C₁-C₄-haloalkyl having 1 to 5 halogen atoms,
or
A represents the radical of the formula (A11) 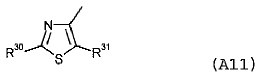 in which
R³⁰ represents hydrogen, halogen, amino, C₁-C₄-alkylamino, di-(C₁-C₄-alkyl)amino, cyano, C₁-C₄-alkyl or C₁-C₄-haloalkyl having 1 to 5 halogen atoms and
R³¹ represents halogen, C₁-C₄-alkyl or C₁-C₄-haloalkyl having 1 to 5 halogen atoms,
or
A represents the radical of the formula (A12) 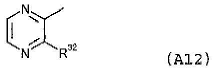 in which
R³² represents hydrogen, halogen, C₁-C₄-alkyl or C₁-C₄-haloalkyl having 1 to 5 halogen atoms,
with the proviso that R³² does not represent chlorine if R¹ and R² simultaneously represent hydrogen,
or
A represents the radical of the formula (A13) 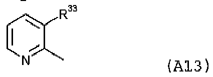 in which
R³³ represents halogen, hydroxyl, C₁-C₄-alkyl, C₁-C₄-alkoxy, C₁-C₄-alkylthio, C₁-C₄-haloalkyl, C₁-C₄-haloalkylthio or C₁-C₄-haloalkoxy having in each case 1 to 5 halogen atoms,
or
A represents the radical of the formula (A14) 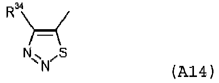 in which
R³⁴ represents C₁-C₄-alkyl.

2. 1,3-Dimethylbutylcarboxanilides of the formula (I) according to Claim 1 in which
R¹ represents hydrogen, C₁-C₆-alkyl, C₁-C₄-alkylsulphinyl, C₁-C₄-alkylsulphonyl, C₁-C₃-alkoxy-C₁-C₃-alkyl, C₃-C₆-cycloalkyl; C₁-C₄-haloalkyl, C₁-C₄-haloalkylthio, C₁-C₄-haloalkylsulphinyl, C₁-C₄-haloalkylsulphonyl, halo-C₁-C₃-alkoxy-C₁-C₃-alkyl, C₃-C₈-halocycloalkyl having in each case 1 to 9 fluorine, chlorine and/or bromine atoms; formyl, formyl-C₁-C₃-alkyl, (C₁-C₃-alkyl)carbonyl-C₁-C₃-alkyl, (C₁-C₃-alkoxy)carbonyl-C₁-C₃-alkyl; halo- (C₁-C₃-alkyl) carbonyl-C₁-C₃-alkyl, halo-(C₁-C₃-alkoxy)carbonyl-C₁-C₃-alkyl having in each case 1 to 13 fluorine, chlorine and/or bromine atoms; or -C(=O) C(=O)R³, -CONR⁴R⁵ or -CH₂NR⁶R⁷,
R² represents hydrogen,
R³ represents hydrogen, C₁-C₆-alkyl, C₁-C₄-alkoxy, C₁-C₃-alkoxy-C₁-C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms,
R⁴ and R⁵ independently of one another represent hydrogen, C₁-C₆-alkyl, C₁-C₃-alkoxy-C₁-C₃-alkyl, C₃-C₆-cycloalkyl; C₁-C₄-haloalkyl, halo-C₁-C₃-alkoxy-C₁-C₃-alkyl, C₃-C₆-halocycloalkyl having in each case 1 to 9 fluorine, chlorine and/or bromine atoms,
R⁴ and R⁵ furthermore together with the nitrogen atom to which they are attached form a saturated heterocycle having 5 or 6 ring atoms which is optionally mono- to tetrasubstituted by identical or different substituents from the group consisting of halogen and C₁-C₄-alkyl, where the heterocycle may contain 1 or 2 further non-adjacent heteroatoms from the group consisting of oxygen, sulphur and NR⁸,
R⁶ and R⁷ independently of one another represent hydrogen, C₁-C₆-alkyl, C₃-C₆-cycloalkyl; C₁-C₄-haloalkyl, C₃-C₆-halocycloalkyl having in each case 1 to 9 fluorine, chlorine and/or bromine atoms,
R⁶ and R⁷ furthermore together with the nitrogen atom to which they are attached form a saturated heterocycle having 5 or 6 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR⁸,
R⁸ represents hydrogen or C₁-C₄-alkyl,
A represents the radical of the formula (A1) 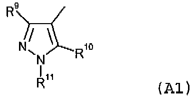 in which
R⁹ represents fluorine, chlorine, bromine or C₂-haloalkyl having 1 to 5 fluorine, chlorine and/or bromine atoms,
R¹⁰ represents hydrogen, chlorine, bromine, iodine, methyl, ethyl, methoxy, ethoxy, methylthio, ethylthio or C₁-C₂-haloalkyl having 1 to 5 halogen atoms,
R¹¹ represents hydrogen, methyl, ethyl, n-propyl, isopropyl, C₁-C₂-haloalkyl having 1 to 5 fluorine, chlorine and/or bromine atoms, hydroxymethyl, hydroxyethyl, cyclopropyl, cyclopentyl, cyclohexyl or phenyl,
or
A represents the radical of the formula (A2) 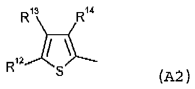 in which
R¹² and R¹³ independently of one another represent hydrogen, fluorine, chlorine, bromine, methyl, ethyl or C₁-C₂-haloalkyl having 1 to 5 fluorine, chlorine and/or bromine atoms,
R¹⁴ represents fluorine, chlorine, bromine, iodine, cyano, methyl, ethyl, C₁-C₂-haloalkyl or C₁-C₂-haloalkoxy having in each case 1 to 5 fluorine, chlorine and/or bromine atoms, with the proviso that R¹⁴ does not represent methyl if R¹² and R¹³ represent hydrogen or methyl and R¹ and R² simultaneously represent hydrogen, or
or
A represents the radical of the formula (A3) 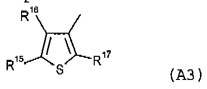 in which
R¹⁵ and R¹⁶ independently of one another represent hydrogen, fluorine, chlorine, bromine, methyl, ethyl or C₁-C₂-haloalkyl having 1 to 5 fluorine, chlorine and/or bromine atoms,
R¹⁷ represents hydrogen, methyl, ethyl or C₁-C₂-haloalkyl having 1 to 5 fluorine, chlorine and/or bromine atoms,
or
A represents the radical of the formula (A4) 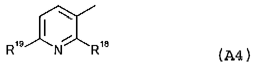 in which
R¹⁸ represents fluorine, chlorine, bromine, iodine, hydroxyl, cyano, C₁-C₄-alkyl, methoxy, ethoxy, methylthio, ethylthio, difluoromethylthio, trifluoromethylthio, C₁-C₂-haloalkyl or C₁-C₂-haloalkoxy having in each case 1 to 5 fluorine, chlorine and/or bromine atoms,
R¹⁹ represents hydrogen, fluorine, chlorine, bromine, iodine, cyano, C₁-C₄-alkyl, methoxy, ethoxy, methylthio, ethylthio, C₁-C₂-haloalkyl or C₁-C₂-haloalkoxy having in each case 1 to 5 fluorine, chlorine and/or bromine atoms C₁-C₂-alkyl sulphinyl or C₁-C₂-alkylsulphonyl, with the proviso,
a) that R¹⁸ does not represent trifluoromethyl, methyl, chlorine or methylthio if R¹⁹ represents hydrogen,
or
A represents the radical of the formula (A5) 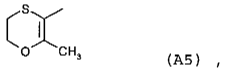 with the proviso that R¹ and R² do not simultaneously represent hydrogen if A represents A5,
or
A represents the radical of the formula (A8) 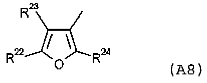 in which
R²² and R²³ independently of one another represent hydrogen, fluorine, Chlorine, bromine, amino, methyl, ethyl or C₁-C₂-haloalkyl having 1 to 5 fluorine, chlorine and/or bromine atoms,
R²⁴ represents hydrogen, methyl, ethyl or C₁-C₂-haloalkyl having 1 to 5 fluorine, chlorine and/or bromine atoms,
or
A represents the radical of the formula (A9) 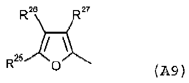 in which
R²⁵ and R²⁶ independently of one another represent hydrogen, fluorine, chlorine, bromine, amino, nitro, methyl, ethyl or C₁-C₂-haloalkyl having 1 to 5 fluorine, chlorine and/or bromine atoms,
R²⁷ represents fluorine, chlorine, bromine, methyl, ethyl or C₁-C₂-haloalkyl having 1 to 5 fluorine, chlorine and/or bromine atoms,
or
A represents the radical of the formula (A11) 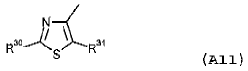 in which
R³⁰ represents hydrogen, fluorine, chlorine, bromine, amino, C₁-C₄-alkylamino, di-(C₁-C₄-alkyl)amino, cyano, methyl, ethyl or C₁-C₂-haloalkyl having 1 to 5 fluorine, chlorine and/or bromine atoms,
R³¹ represents fluorine, chlorine, bromine, methyl, ethyl or C₁-C₂-haloalkyl having 1 to 5 fluorine, chlorine and/or bromine atoms,
or
A represents the radical of the formula (A12) 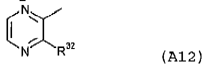 in which
R³² represents hydrogen, fluorine, chlorine, bromine, methyl, ethyl or C₁-C₂-haloalkyl having 1 to 5 fluorine, chlorine and/or bromine atoms,
with the proviso that R³² does not represent chlorine if R¹ and R² simultaneously represent hydrogen,
or
A reresents the radical of the formula (A13) 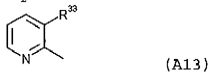 in which
R³³ represents fluorine, chlorine, bromine, iodine, hydroxyl, C₁-C₄-alkyl, methoxy, ethoxy, methylthio, ethylthio, difluoromethylthio, trifluoromethylthio, C₁-C₂-haloalkyl or C₁-C₂-haloalkoxy having in each case 1 to 5 fluorine, chlorine and/or bromine atoms.

3. 1,3-Dimethylbutylcarboxanilides of the formula (I) according to Claim 1 or 2 in which R¹ represents formyl.

4. 1,3-Dimethylbutylcarboxanilides of the formula (I) according to Claim 1 or 2 in which R¹ represents -C(=O)C(=O)R³, where R³ is as defined in Claim 1 or 2.

5. 1,3-Dimethylbutylcarboxanilides of the formula (I) according to Claim 1 or 2 in which A represents A1.

6. Process for preparing compounds of the formula (I) according to Claim 1, **characterized in that** a) carboxylic acid derivatives of the formula (II) 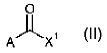 in which
A is as defined in Claim 1 and
X¹ represents halogen or hydroxyl, are reacted with aniline derivatives of the formula (III) 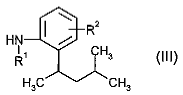 in which R¹ and R² are as defined in Claim 1, if appropriate in the presence of a catalyst, if appropriate in the prescence of a condensing agent, if appropriate in the presence of an acid binder and if appropriate in the presence of a diluent, or
b) hexylcarboxanilides of the formula (I-a) 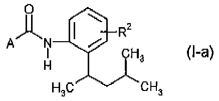 in which A and R² are as defined in Claim 1, are reacted with halides of the formula (IV)
R^{1-A}-X² (IV)
in which
X² represents chlorine, bromine or iodine,
R¹ represents C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C₄-alkyl, C₃-C₈-halocycloalkyl having in each case 1 to 9 fluorine, chlorine and/or bromine atoms; formyl, formyl-C₁-C₃-alkyl, (C₁-C₃-alkyl) carbonyl-C₁-C₃-alkyl, (C₁-C₃-alkoxy)carbonyl-C₁-C₃-alkyl; halo-(C₁-C₃-alkyl)carbonyl-C₁-C₃-alkyl, halo- (C₁-C₃-alkoxy)carbonyl-C₁-C₃-alkyl having in each case 1 to 13 fluorine, chlorine and/or bromine atoms; or -C(=O)C(=O)R³, CONR⁴R⁵ or -CH₂NR⁶R⁷,
where R³, R⁴, R⁵, R⁶ and R⁷ are as defined in Claim 1, in the presence of a base and in the presence of a diluent.

7. Compositions for controlling unwanted microorganisms, **characterized in that** they comprise at least one 1,3-dimethylbutylcarboxanilide of the formula (I) according to Claim 1 in addition to extenders and/or surfactants.

8. Use of 1,3-dimethylbutylcarboxanilides of the formula (I) according to Claim 1 for controlling unwanted microorganisms in crop protection, in the protection of materials and in the treatment of seeds.

9. Method for controlling unwanted microorganisms in crop protection, in the protection of materials and in the treatment of seeds, **characterized in that** 1,3-dimethylbutylcarboxanilides of the formula (I) according to Claim 1 are applied to the microorganisms and/or their habitat.

10. Process for preparing compositions for controlling unwanted microorganisms, **characterized in that** 1,3-dimethylbutylcarboxanilides of the formula (I) according to Claim 1 are mixed with extenders and/or surfactants.

## Revendications

1. 1,3-Diméthylbutylcarboxanilides de formule (I) 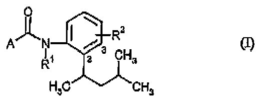 dans laquelle
R¹ représente hydrogène, alkyle en C₁-C₈, alkylsulfinyle en C₁-C₆, alkylsulfonyle en C₁-C₆, alcoxy en C₁-C₄-alkyle en C₁-C₄, cycloalkyle en C₃-C₈ ; halogénoalkyle en C₁-C₆, halogénoalkylthio en C₁-C₄, halogénoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃) carbonyl-alkyle en C₁-C₃, (alcoxy en C₁-C₃) carbonyl-alkyle en C₁-C₃ ; halogéno-(alkyle en C₁-C₃) carbonyl-alkyle en C₁-C₃, halogéno-(alcoxy en C₁-C₃) carbonyl-alkyle en C₁-C₃ contenant à chaque fois 1 à 13 atomes de fluor, de chlore et/ou de brome ;
ou -C(=O) C(=O)R³, -CONR⁴R⁵ ou -CH₂NR⁶R⁷,
R² représente hydrogène,
R³ représente hydrogène, alkyle en C₁-C₈, alcoxy en C₁-C₈, alcoxy en C₁-C₄-alkyle en C₁-C₄, cycloalkyle en C₃-C₈ ; halogénoalkyle en C₁-C₆, halogénoalcoxy en C₁-C₆, halogéno-alcoxy en C₁-C₄-alkyle en C₁-C₄, halogénocycloalkyle en C₃-C₈ contenant à chaque fois 1 à 9 atomes de fluor, de chlore et/ou de brome,
R⁴ et R⁵ représentent chacun indépendamment l'un de l'autre hydrogène, alkyle en C₁-C₈, alcoxy en C₁-C₄-alkyle en C₁-C₄, cycloalkyle en C₃-C₈; halogênoalkyle en C₁-C₈, halogéno-alcoxy en C₁-C₄-alkyle en C₁-C₄, halogénocycloalkyle en C₃-C₈ contenant à chaque fois 1 à 9 atomes de fluor, de chlore et/ou de brome,
R⁴ et R⁵ peuvent également former ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR⁸,
R⁶ et R⁷ représentent indépendamment l'un de l'autre hydrogène, alkyle en C₁-C₈, cycloalkyle en C₃-C₈; halogénoalkyle en C₁-C₈, halogénocycloalkyle en C₃-C₈ contenant à chaque fois 1 à 9 atomes de fluor, de chlore et/ou de brome,
R⁶ et R⁷ peuvent également former ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR⁸,
R⁸ représente hydrogène ou alkyle en C₁-C₆,
A représente le radical de formule (A1) 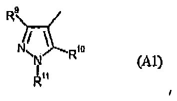 dans laquelle
R⁹ représente fluor, chlore, brome, ou halogénoalkyle en C₂ contenant 1 à 5 atomes d'halogène,
R¹⁰ représente hydrogène, chlore, brome, iode, cyano, alkyle en C₁-C₄, alcoxy en C₂-C₄, alkylthio en C₁-C₄ ou halogénoalkyle en C₁-C₄ contenant 1 à 5 atomes d'halogène,
R¹¹ représente hydrogène, alkyle en C₁-C₄, hydroxy-alkyle en C₁-C₄, alcényle en C₂-C₆, cycloalkyle en C₃-C₆, alkylthio en C₁-C₄-alkyle en C₁-C₄, alcoxy en C₁-C₄-alkyle en C₁-C₄ ; halogénoalkyle en C₁-C₄, halogénoalkylthio en C₁-C₄-alkyle en C₁-C₄, halogénoalcoxy en C₁-C₄-alkyle en C₁-C₄ contenant à chaque fois 1 à 5 atomes d'halogène, ou phényle,
ou
A représente le radical de formule (A2) 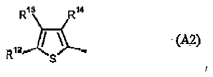 dans laquelle
R¹² et R¹³ représentent indépendamment l'un de l'autre hydrogène, halogène, alkyle en C₁-C₄ ou halogénoalkyle en C₁-C₄ contenant à chaque fois 1 à 5 atomes d'halogène, et
R¹⁴ représente halogène, cyano ou alkyle en C₁-C₄, ou halogénoalkyle en C₁-C₄ ou halogënoalcoxy en C₁-C₄ contenant à chaque fois 1 à 5 atomes d'halogène,
à condition que R¹⁴ ne représente pas méthyle lorsque R¹² et R¹³ représentent hydrogène ou méthyle et R¹ et R² représentent simultanément hydrogène,
ou
A représente le radical de formule (A3) 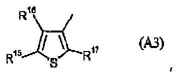 dans laquelle
R¹⁵ et R¹⁶ représentent indépendamment l'un de l'autre hydrogène, halogène, alkyle en C₁-C₄ ou halogénoalkyle en C₁-C₄ contenant 1 à 5 atomes d'halogène, et
R¹⁷ représente hydrogène, alkyle en C₁-C₄ ou
halogénoalkyle en C₁-C₄ contenant 1 à 5 atomes d'halogène,
ou
A représente le radical de formule (A4) 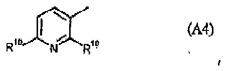 dans laquelle R¹⁸ représente halogène, hydroxy, cyano, alkyle en C₁-C₄, alcoxy en C₁-C₄, alkylthio en C₁-C₄, halogénoalkyle en C₁-C₄, halogénoalkylthio en C₁-C₄ ou halogénoalcoxy en C₁-C₄ contenant à chaque fois 1 à 5 atomes d'halogène, R¹⁹ représente hydrogène, halogène, cyano, alkyle en C₁-C₄, alcoxy en C₁-C₄, alkylthio en C₁-C₄, halogénoalkyle en C₁-C₄, halogénoalcoxy en C₁-C₄ contenant à chaque fois 1 à 5 atomes d'halogène, alkylsulfinyle en C₁-C₄ ou alkylsulfonyle en C₁-C₄,
à condition que
a) R¹⁸ ne représente pas trifluorométhyle, méthyle, chlore ou méthylthio lorsque R¹⁹ représente hydrogène et R¹ et R² représentent simultanément hydrogène,
ou
A représente le radical de formule (A5) 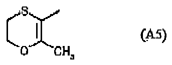 à condition que R¹ et R² ne représentent pas simultanément hydrogène lorsque A représente A5,
ou
A représente le radical de formule (A6) 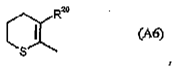 dans laquelle R²⁰ représente alkyle en C₁-C₄ ou halogénoalkyle en C₁-C₄ contenant 1 à 5 atomes d'halogène,
ou
A représente le radical de formule (A7) 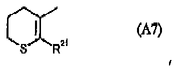 dans laquelle R²¹ représente alkyle en C₁-C₄ ou halogënoalkyle en C₁-C₄ contenant 1 à 5 atomes d'halogène,
ou
A représente le radical de formule (A8) 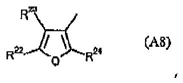 dans laquelle R²² et R²³ représentent indépendamment l'un de l'autre hydrogène, halogène, amino, alkyle en C₁-C₄ ou halogénoalkyle en C₁-C₄ contenant 1 à 5 atomes d'halogène, et
R²⁴ représente hydrogène, alkyle en C₁-C₄ ou halogénoalkyle en C₁-C₄ contenant 1 à 5 atomes d'halogène,
à condition que R²⁴ ne représente pas méthyle lorsque R²² et R²³ représentent hydrogène ou méthyle et R¹ et R² représentent simultanément hydrogène,
ou
A représente le radical de formule (A9) 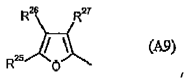 dans laquelle
R²⁵ et R²⁶ représentent indépendamment l'un de l'autre hydrogène, halogène, amino, nitro, alkyle en C₁-C₄ ou halogénoalkyle en C₁-C₄ contenant 1 à 5 atomes d'halogène, et
R²⁷ représente halogène, alkyle en C₁-C₄ ou halogénoalkyle en C₁-C₄ contenant 1 à 5 atomes d'halogène,
ou
A représente le radical de formule (A11) 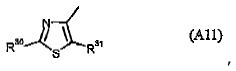 dans laquelle
R³⁰ représente hydrogène, halogène, amino, alkylamino en C₁-C₄, di-(alkyle en C₁-C₄)-amino, cyano, alkyle en C₁-C₄ ou halogénoalkyle en C₁-C₄ contenant 1 à 5 atomes d'halogène, et
R³¹ représente halogène, alkyle en C₁-C₄ ou halogénoalkyle en C₁-C₄ contenant 1 à 5 atomes d'halogène,
ou
A représente le radical de formule (A12) 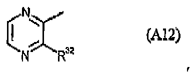 dans laquelle
R³² représente hydrogène, halogène, alkyle en C₁-C₄ ou halogénoalkyle en C₁-C₄ contenant 1 à 5 atomes d'halogène,
à condition que R³² ne représente pas chlore lorsque R¹ et R² représentent simultanément hydrogène,
ou
A représente le radical de formule (A13) 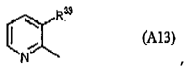 dans laquelle
R³³ représente halogène, hydroxy, alkyle en C₁-C₄, alcoxy en C₁-C₄, alkylthio en C₁-C₄, halogénoalkyle en C₁-C₄, halogénoalkylthio en C₁-C₄ ou halogénoalcoxy en C₁-C₄ contenant à chaque fois 1 à 5 atomes d'halogène, ou
A représente le radical de formule (A14) 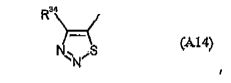 dans laquelle R³⁴ représente alkyle en C₁-C₄.

2. 1,3-Diméthylbutylcarboxanilides de formule (I) selon la revendication 1, dans lesquels R¹ représente hydrogène, alkyle en C₁-C₆, alkylsulfinyle en C₁-C₄, alkylsulfonyle en C₁-C₄, alcoxy en C₁-C₃-alkyle en C₁-C₃, cycloalkyle en C₃-C₆ ; halogénoalkyle en C₁-C₄, halogénoalkylthio en C₁-C₄, halogénoalkylsulfinyle en C₁-C₄, halogénoalkylaulfonyle en C₁-C₄, halogéno-alcoxy en C₁-C₃-alkyle en C₁-C₃, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃)carbonyl-alkyle en C₁-C₃, (alcoxy en C₁-C₃) carbonyl-alkyle en C₁-C₃; halogéno-(alkyle en C₁-C₃) carbonyl-alkyle en C₁-C₃, halogéno-(alcoxy en C₁-C₃) carbonyl-alkyle en C₁-C₃ contenant à chaque fois 1 à 13 atomes de fluor, de chlore et/ou de brome ;
ou -C(=O)C(=O)R³, -CONR⁴R⁵ ou -CH₂NR⁶R⁷,
R² représente hydrogène,
R³ représente hydrogène, alkyle en C₁-C₆, alcoxy en C₁-C₄, alcoxy en C₁-C₃-alkyle en C₁-C₃, cycloalkyle en C₃-C₆ ; halogénoalkyle en C₁-C₄, halogénoalcoxy en C₁-C₄, halogéno-alcoxy en C₁-C₃-alkyle en C₁-C₃, halogènocycloalkyle en C₃-C₆ contenant à chaque fois 1 à 9 atomes de fluor, de chlore et/ou de brome,
R⁴ et R⁵ représentent chacun indépendamment l'un de l'autre hydrogène, alkyle en C₁-C₆, alcoxy en C₁-C₃-alkyle en C₁-C₃, cycloalkyle en C₃-C₆ ; halogénoalkyle en C₁-C₄, halogéno-alcoxy en C₁-C₃-alkyle en C₁-C₃, halogénocycloalkyle en C₃-C₆ contenant à chaque fois 1 à 9 atomes de fluor, de chlore et/ou de brome,
R⁴ et R⁵ peuvent également former ensemble avec l'atome d'azote auquel ils sont reliés un hétérocycle saturé de 5 ou 6 atomes de cycle, éventuellement substitué une à quatre fois, de manière identique ou différente, par halogène ou alkyle en C₁-C₄, l'hétérocycle pouvant contenir 1 ou 2 hétéroatomes non voisins supplémentaires de la série oxygène, soufre ou NR⁸,
R⁶ et R⁷ représentent indépendamment l'un de l'autre hydrogène, alkyle en C₁-C₆, cycloalkyle en C₃-C₆ ; halogénoalkyle en C₁-C₄, halogénocycloalkyle en C₃-C₆ contenant à chaque fois 1 à 9 atomes de fluor, de chlore et/ou de brome,
R⁶ et R⁷ peuvent également former ensemble avec l'atome d'azote auquel ils sont reliés un hétérocycle saturé de 5 ou 6 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série oxygène, soufre ou NR⁸,
R⁸ représente hydrogène ou alkyle en C₁-C₄,
A représente le radical de formule (A1) 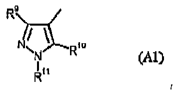 dans laquelle
R⁹ représente fluor, chlore, brome ou halogénoalkyle en C₂ contenant 1 à 5 atomes de fluor, de chlore et/ou de brome,
R¹⁰ représente hydrogène, chlore, brome, iode, méthyle, éthyle, méthoxy, éthoxy, méthylthio, éthylthio ou halogénoalkyle en C₁-C₂ contenant 1 à 5 atomes d'halogène,
R¹¹ représente hydrogène, méthyle, éthyle, n-propyle, iso-propyle, halogénoalkyle en C₁-C₂ contenant 1 à 5 atomes de fluor, de chlore et/ou de brome, hydroxyméthyle, hydroxyéthyle, cyclopropyle, cyclopentyle, cyclohexyle ou phényle,
ou
A représente le radical de formule (A2) 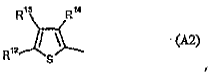 dans laquelle R¹² et R¹³ représentent indépendamment l'un de l'autre hydrogène, fluor, chlore, brome, méthyle, éthyle ou halogénoalkyle en C₁-C₂ contenant 1 à 5 atomes de fluor, de chlore et/ou de brome,
R¹⁴ représente fluor, chlore, brome, iode, cyano, méthyle, éthyle, halogénoalkyle en C₁-C₂ ou halogénoalcoxy en C₁-C₂ contenant à chaque fois 1 à 5 atomes de fluor, de chlore et/ou de brome, condition que R¹⁴ ne représente pas méthyle lorsque R¹² et R¹³ représentent hydrogène ou méthyle et R¹ et R² représentent simultanément hydrogène,
ou
A représente le radical de formule (A3) 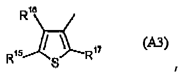 dans laquelle R¹⁵ et R¹⁶ représentent indépendamment l'un de l'autre hydrogène, fluor, chlore, brome, méthyle, éthyle ou halogénoalkyle en C₁-C₂ contenant 1 à 5 atomes de fluor, de chlore et/ou de brome, et
R¹⁷ représente hydrogène, méthyle, éthyle ou halogénoalkyle en C₁-C₂ contenant 1 à 5 atomes de fluor, de chlore et/ou de brome,
ou
A représente le radical de formule (A4) 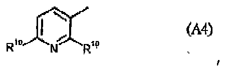 dans laquelle
R¹⁸ représente fluor, chlore, brome, iode, hydroxy, cyano, alkyle en C₁-C₄, méthoxy, éthoxy, methylthio, éthylthio, difluorométhylthio, trifluorométhylthio, halogénoalkyle en C₁-C₂ ou halogénoalcoxy en C₁-C₂ contenant à chaque fois 1 à 5 atomes de fluor, de chlore et/ou de brome,
R¹⁹ représente hydrogène, fluor, chlore, brome, iode,
cyano, alkyle en C₁-C₄, méthoxy, éthoxy, méthylthio, éthylthio, halogénoalkyle en C₁-C₂ ou halogénoalcoxy en C₁-C₂ contenant à chaque fois 1 à 5 atomes de fluor, de chlore et/ou de brome, alkylsulfinyle en C₁-C₂ ou alkylsulfonyle en C₁-C₂,
à condition que
a) R¹⁸ ne représente pas trifluorométhyle, méthyle, chlore ou méthylthio lorsque R¹⁹ représente hydrogène, ou
A représente le radical de formule (A5) 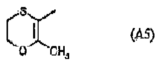 à condition que R¹ et R² ne représentent pas simultanément hydrogène lorsque A représente A5,
ou
A représente le radical de formule (A8) 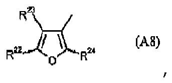 dans laquelle
R²² et R²³ représentent indépendamment l'un de l'autre hydrogène, fluor, chlore, brome, amino, méthyle, éthyle ou halogénoalkyle en C₁-C₂ contenant 1 à 5 atomes de fluor, de chlore et/ou de brome,
R²⁴ représente hydrogène, méthyle, éthyle ou halogénoalkyle en C₁-C₂ contenant 1 à 5 atomes de fluor, de chlore et/ou de brome,
ou
A représente le radical de formule (A9) 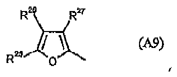 dans laquelle R²⁵ et R²⁶ représentent indépendamment l'un de l'autre hydrogène, fluor, chlore, brome, amino, nitro, méthyle, éthyle ou halogénoalkyle en C₁-C₂ contenant 1 à 5 atomes de fluor, de chlore et/ou de brome,
R²⁷ représente fluor, chlore, brome, méthyle, éthyle ou halogénoalkyle en C₁-C₂ contenant 1 à 5 atomes de fluor, de chlore et/ou de brome,
ou
A représente le radical de formule (A11) 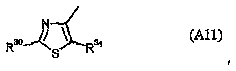 dans laquelle
R³⁰ représente hydrogène, fluor, chlore, brome, amino, alkylamino en C₁-C₄, di-(alkyle en C₁-C₄)-amino, cyano, méthyle, éthyle ou halogénoalkyle en C₁-C₂ contenant 1 à 5 atomes de fluor, de chlore et/ou de brome,
R³¹ représente fluor, chlore, brome, méthyle, éthyle ou halogénoalkyle en C₁-C₂ contenant 1 à 5 atomes de fluor, de chlore et/ou de brome,
ou
A représente le radical de formule (A12) 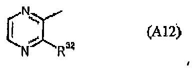 dans laquelle
R³² représente hydrogène, fluor, chlore, brome, méthyle, éthyle ou halogénoalkyle en C₁-C₂ contenant 1 à 5 atomes de fluor, de chlore et/ou de brome,
à condition que R³² ne représente pas le chlore lorsque R¹ et R² représentent simultanément l'hydrogène,
ou
A représente le radical de formule (A13) 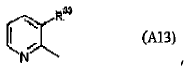 dans laquelle R³³ représente fluor, chlore, brome, iode, hydroxy, alkyle en C₁-C₄, méthoxy, éthoxy, méthylthio, éthylthio, difluorométhylthio, trifluorométhylthio, halogénoalkyle en C₁-C₂ ou halogénoalcoxy en C₁-C₂ contenant à chaque fois 1 à 5 atomes de fluor, de chlore et/ou de brome.

3. 1,3-Diméthylbutylcarboxanilides de formule (I) selon la revendication 1 ou 2, dans lesquels R¹ représente formyle.

4. 1,3-Diméthylbutylcarboxanilides de formule (I) selon la revendication 1 ou 2, dans lesquels R¹ représente -C(=O)C(=O)R³, R³ ayant les significations indiquées dans la revendication 1 ou 2.

5. 1,3-Diméthylbutylcarboxanilides de formule (I) selon la revendication 1 ou 2, dans lequel A représente Al.

6. Procédé de fabrication des composés de formule (I) selon la revendication 1, **caractérisé en ce que** a) des dérivés d'acides carboxyliques de formule (II) 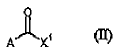 dans laquelle
A a les significations indiquées dans la revendication 1 et
X¹ représente halogène ou hydroxy,
sont mis en réaction avec des dérivés d'aniline de formule (III) 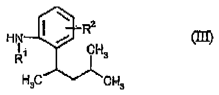 dans laquelle R¹ et R² ont les significations indiquées dans la revendication 1,
éventuellement en présence d'un catalyseur,
éventuellement en présence d'un agent de condensation, éventuellement en présence d'un liant d'acide et éventuellement en présence d'un diluant,
ou
b) des hexylcarboxanilides de formule (I-a) 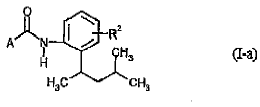 dans laquelle A et R² ont les significations indiquées dans la revendication 1,
sont mis en réaction avec des halogénures de formule (IV)
R^{1-A}-X² (IV)
dans laquelle
X² représente chlore, brome ou iode,
R¹ représente alkyle en C₁-C₈, alkylsulfinyle en C₁-C₆, alkylsulfonyle en C₁-C₆, alcoxy en C₁-C₄-alkyle en C₁-C₄, cycloalkyle en C₃-C₈; halogénoalkyle en C₁-C₆, halogënoalkylthio en C₁-C₄, halogënoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, de chlore et/ou de brome ; formyle, formyl-alkyle en C₁-C₃, (alkyle en C₁-C₃)carbonyl-alkyle en C₁-C₃, (alcoxy en C₁-C₃)carbonyl-alkyle en C₁-C₃; halogéno-(alkyle en C₁-C₃)carbonyl-alkyle en C₁-C₃, halogéno-(alcoxy en C₁-C₃)carbonyl-alkyle en C₁-C₂ contenant à chaque fois 1 à 13 atomes de fluor, de chlore et/ou de brome ;
ou -C(=O)C(=O)R³, CONR⁴R⁵ ou -CH₂NR⁶R⁷, R³, R⁴, R⁵, R⁶ et R⁷ ayant les significations indiquées dans la revendication 1,
en présence d'une base et en présence d'un diluant.

7. Agent de lutte contre les microorganismes indésirables, **caractérisé par** une teneur en au moins un 1,3-diméthylbutylcarboxanilide de formule (I) selon la revendication 1 en plus d'extendeurs et/ou de substances tensioactives.

8. Utilisation de 1,3-diméthylbutylcarboxanilides de formule (I) selon la revendication 1 pour lutter contre les microorganismes indésirables dans le cadre de la protection des plantes, de la protection des matériaux et du traitement des graines.

9. Procédé de lutte contre les microorganismes indésirables dans le cadre de la protection des plantes, de la protection des matériaux et du traitement des graines, **caractérisé en ce que** des 1,3-diméthylbutylcarboxanilides de formule (I) selon la revendication 1 sont appliqués sur les microorganismes et/ou leur habitat.

10. Procédé de fabrication d'agents pour lutter contre les microorganismes indésirables, **caractérisé en ce que** des 1,3-diméthylbutylcarboxanilides de formule (I) selon la revendication 1 sont mélangés avec des extendeurs et/ou des substances tensioactives.
